# EUROPEAN PATENT SPECIFICATION

(11) **EP 4 027 871 B1**
(45) Date of publication and mention of the grant of the patent: **18.02.2026**
(21) Application number: 20863986.4
(22) Date of filing: 11.09.2020
(51) Int. Cl.: A61B 5/00, A61B 8/02, A61B 5/024, A61B 5/1464, A61B 5/08, A61B 5/021, A61B 5/0205, A61B 5/11, A61B 5/1455, A61B 5/113, A61B 8/08

(54) **MULTIPLEXED WEARABLE SENSORS FOR PREGNANCY MONITORING**
AM KÖRPER TRAGBARE MULTIPLEXIERTE SENSOREN ZUR SCHWANGERSCHAFTSÜBERWACHUNG
CAPTEURS PORTABLES MULTIPLEXÉS POUR SURVEILLANCE DE GROSSESSE

(30) Priority: 11.09.2019 US 201962898983 P
(43) Date of publication of application: 20.07.2022
(73) Proprietor: Northwestern University, Evanston Illinois 60208-1111 (US)
(72) Inventor: XU, Shuai, Chicago, Illinois 60611 (US); CHUNG, Ha Uk, Evanston, Illinois 60201 (US); JEONG, Hyoyoung, Evanston, Illinois 60201 (US); KIM, Dong Hyun, Evanston, Illinois 60201 (US); LEE, Jong Yoon, Morton Grove, Illinois 60053 (US); RYU, Dennis, Evanston, Illinois 60201 (US); ROGERS, John A., Wilmette, Illinois 60091 (US)
(74) Representative: Weickmann & Weickmann PartmbB
(86) International application number: PCT/US2020/050316
(87) International publication number: WO 2021/050818

(56) References cited:
- WO-A1-2018/013569
- WO-A1-2019/133926
- WO-A1-2019/133926
- WO-A1-2019/161277
- WO-A2-00/54650
- US-A1- 2016 004 224
- US-A1- 2016 004 224
- US-A1- 2016 058 363
- US-A1- 2018 133 105
- ANONYMOUS: "Bluetooth Low Energy - Wikipedia", 5 November 2019 (2019-11-05), XP093232615, Retrieved from the Internet <URL:https://en.wikipedia.org/w/index.php?title=Bluetooth_Low_Energy&oldid=914166074>

## Description

### FIELD OF THE INVENTION

The invention relates generally to biosensors, and more particularly, to a battery-powered, Bluetooth enabled vital signs monitoring system that exploits a network of wearable, wireless sensors that are able to recapitulate a full suite of maternal/fetal vital signs.

### BACKGROUND OF THE INVENTION

The background description provided herein is for the purpose of generally presenting the context of the invention. The subject matter discussed in the background of the invention section should not be assumed to be prior art merely as a result of its mention in the background of the invention section. Similarly, a problem mentioned in the background of the invention section or associated with the subject matter of the background of the invention section should not be assumed to have been previously recognized in the prior art. The subject matter in the background of the invention section merely represents different approaches, which in and of themselves may also be inventions.

Pregnancy is a high acuity health state experienced by more than 200 million women annually. Though pregnancy, labor and delivery are common events, there remains a continued risk of catastrophic outcomes with 23.8 deaths occurring per 100,000 births in the U.S. and 462 deaths occurring per 100,000 births in low income countries. Each year, approximately 295,000 women die as a result of child birth complications with 94% of these deaths occurring in low- and middle-income countries (LMICs). The underlying drivers of maternal morbidity are both preventable and predictable - the most common causes of hemorrhage, hypertensive disorders, infection and sepsis are all preceded by predictable perturbations of vital signs across both high resource and low resource settings. The timely identification and monitoring of pregnant women for these women is the critical first step to appropriate intervention.

However, standard monitoring systems have exhibited minimal change over the past 30 years. Currently, large rigid monitoring devices are affixed to a women's abdomen with accompanying wires that tether to base units. Furthermore, pregnancy monitoring is largely limited to intrapartum monitoring of the fetus within a hospital with additional monitoring equipment required in instances of maternal or fetal distress such as pulse oximetry, blood pressure monitors, and electrocardiography. Often, these systems operate in a non-continuous manner requiring high provider burden. The failure to recognize and act on deteriorating vital signs is recognized as a key driver of avoidable maternal deaths. Furthermore, the high base costs, expensive consumables, and operational burden of existing monitoring systems often preclude deployment in LMIC settings where maternal morbidity is the highest. In many parts of the world, a partograph, a paper form, remains the only available method to track labor in spite of a lack of evidence for clinical benefit for laboring women.

Therefore, there is a critical need for new classes of advanced maternal monitoring systems that offer comprehensive and continuous clinical-grade vital signs measurements, advanced monitoring capabilities to support clinical decision making, and operability in both high resource and low resource settings.

Document WO 2019/133926 discloses systems, devices, and methods for performing trans-abdominal fetal oximetry and/or trans-abdominal fetal pulse oximetry using independent component analysis.

### SUMMARY

In one aspect, the invention relates to a sensor network for pregnancy monitoring of a subject. In one embodiment, the subject is a pregnant woman.

The sensor network includes a plurality of sensor systems time-synchronized to each other. Each sensor system is placed on a respective region of the subject and comprises a sensor member configured to detect data associated with at least one of physiological parameters of the subject, and a Bluetooth low energy system-on-a-chip (BLE SoC) configured to process and transmit the detected data. In one embodiment, the physiological parameters comprise one or more of a maternal heart rate, a respiratory rate, a core body temperature, a peripheral temperature, a blood oxygenation, a blood pressure, uterine contraction frequency and intensity, and a fetal hear rate.

In one embodiment, the plurality of sensor systems comprises a first sensor system, a second sensor system and a third sensor system respectively placed on an abdominal region, a chest region and a limb region of the subject. The limb region can be a finger, toe, foot, wrist, forehead, or ear.

In one embodiment, the second sensor system is configured to be a central node, while the first and third sensor systems are configured to be peripheral nodes.

In one embodiment, the first sensor system is configured to detect a fetal heart rate (FHR), uterine contraction, and/or a maternal heart rate.

In one embodiment, the sensor member of the first sensor system comprises an onboard wireless Doppler ultrasound (US) device for continuously acoustic detection of the FHR.

In one embodiment, the first sensor system further comprises a piezoelectric transducer incorporated as a transmitter and receiver for a Doppler effect. In one embodiment, the piezoelectric transducer is tuned to resonate at about 3 MHz.

In one embodiment, the sensor member of the first sensor system further comprises an electromyograph (EMG) sensor for detection of the uterine contraction frequency and intensity, and/or electrocardiograph (ECG) sensor for simultaneously detection of the fetal ECG and the maternal ECG. In one embodiment, the ECG sensor has at least two electrodes for ECG generation, wherein the at least two electrodes are adjustable in spacing.

In one embodiment, the second sensor system is configured to detect a maternal heart rate, a core body temperature, and/or a respiratory rate (RR).

In one embodiment, the sensor member of the second sensor system comprises one or more of a motion-sensing unit for detecting seismocardiogram (SCG) and chest wall movements for respiratory rate (RR); a thermometer for detecting the core body temperature; and an ECG sensor for detecting the maternal heart rate. In one embodiment, the BLE SoC is configured to operably control the motion-sensing unit through one of a serial peripheral interface (SPI) communication protocol and an inter-integrated circuit (I²C) communication protocol, and to operably control the thermometer through the other of the SPI and the I²C communication protocols.

In one embodiment, the motion-sensing unit comprises a 3-axial accelerometer, and/or an inertial measurement unit (IMU).

In one embodiment, the accelerometer or the IMU is used with a motion artifact module to identify a vital sign as being subject to motion artifact and to correct of motion artifact.

In one embodiment, the third sensor system is configured to detect photoplethysmogram (PPG) and/or a peripheral temperature.

In one embodiment, the sensor member of the third sensor system comprises a pulse oximetry for detecting blood oxygenation (SpO₂) and a thermometer for detecting the peripheral temperature.

In one embodiment, the pulse oximetry comprises a light emitted diode (LED) and a photodetector.

Each of the plurality of sensor systems further comprises a power module coupled to the BLE SoC for providing power to said sensor system.

In one embodiment, the power module comprises a power management integrated circuit (PMIC) coupled to the BLE SoC, and a battery coupled to the PMIC for providing power to said sensor system. In one embodiment, the battery is a rechargeable battery.

In one embodiment, the power module further comprises a wireless charging module coupled to the PMIC for wirelessly charging the rechargeable battery.

In one embodiment, the wireless charging module comprises a near-field communication (NFC) antenna.

In one embodiment, the power module further comprises a failure prevention element including a short-circuit protection component or a circuit to avoid battery malfunction.

In one embodiment, each of the plurality of sensor systems is an epidermal electronic system (EES).

Each of the plurality of sensor systems further comprises a plurality of flexible and stretchable interconnects electrically connecting to a plurality of electronic components including the sensor member, the BLE SoC and the power module; and an elastomeric encapsulation layer at least partially surrounding the electronic components and the flexible and stretchable interconnects to form a tissue-facing surface attached to the subject and an environment-facing surface, wherein the tissue-facing surface is configured to conform to a skin surface of the pregnant woman.

Each of the plurality of sensor systems further comprises one or more foldable electronic boards, wherein the plurality of electronic components and the plurality of flexible and stretchable interconnects are disposed on the one or more foldable electronic boards.

The encapsulation layer is formed of a flame retardant material.

The elastomeric encapsulation layer is a waterproof and biocompatible silicone enclosure.

In one embodiment, each of the first and second sensor systems further comprises a biocompatible hydrogel adhesive for attaching said sensor system on the respective region of the subject, wherein the biocompatible hydrogel adhesive is adapted such that signals from the subject are operably conductible to said sensor system.

The sensor network also includes a controller adapted in wireless communication with the plurality of sensor systems and configured to receive, from the plurality of sensor systems, to process, and to display the physiological parameters.

In one embodiment, the controller is configured to perform at least one function of receiving and processing the detected data of the physiological parameters from the plurality of sensor systems; displaying the processed data of the physiological parameters in real time; transmitting the processed data of the physiological parameters to at least one of a patient database, a cloud server, and a mobile device; continuously monitoring one or more critical parameters associating at least one vital sign; and notifying a practitioner or caregiver when a sensor aberrant signal output condition occurs; and generating an alarm when an alarming vital sign reading condition in which the one or more of the critical parameters are out of pre-defined ranges occurs, and notifying a practitioner or caregiver of the alarm.

In one embodiment, the one or more critical parameters are one or more of the heart parameters, temperature, body movements, respiratory parameters, oxygenation, and blood pressure.

In one embodiment, said processing the detected data of the physiological parameters comprises processing the output signals of the second and third sensor systems to determine a pulse arrival time (PAT) and a pulse transit time (PTT) so as to obtain the blood pressure; eliminating the maternal ECG from the outputs of the first sensor system allowing for fetal ECG isolation; and/or processing the output signals of the first and second sensor systems to subtract motion artifact in physiological signal calculations for respiratory rate and/or heart rate.

In one embodiment, the controller is configured to further perform at least one function of assessing the physiological signs of the fetus and the mother during labor, based on the detected physiological parameters; assessing the physiological signs of the fetus and the mother for women with high-risk obstetrical complications prior to labor; and predicting for the onset of true labor from false labor.

In one embodiment, the controller comprises a mobile device operable serving as a base station for Bluetooth communication.

In one embodiment, the mobile device in bi-directionally wireless communication with the plurality of sensor systems.

In one embodiment, the mobile device is a hand-held device or a portable device having a graphical user interface to display the physiological parameters.

In another aspect, the invention relates to a method for pregnancy monitoring of a subject. The subject is a pregnant woman. The method comprises deploying the sensor network comprising a plurality of sensor systems, wherein each of the plurality of sensor systems comprises a sensor member configured to detect a vital sign of the subject and generate a corresponding one of the physiological parameters, wherein the plurality of sensor systems comprises a first sensor system, a second sensor system and a third sensor system respectively placed on an abdominal region, a chest region and a limb region of the subject; synchronizing the plurality of sensor systems to a common time base; detecting data of the physiological parameters from the plurality of sensor systems; processing the detected data of the physiological parameters from the plurality of sensor systems; displaying the processed data of the physiological parameters in real time; and transmitting the processed data of the physiological parameters to at least one of a patient database, a cloud server, and a mobile device. The limb region can be a finger, toe, foot, wrist, forehead, or ear.

In one embodiment, the first sensor system is configured to detect the FHR, the uterine contraction, and/or the maternal heart rate, the second sensor system is configured to detect the maternal heart rate, the core body temperature, and/or the RR, and the third sensor system is configured to detect the PPG and/or the peripheral temperature.

In one embodiment, said processing the detected data of the physiological parameters comprises processing the output signals of the second and third sensor systems to determine a PAT and a PTT so as to obtain the blood pressure; eliminating the maternal ECG from the outputs of the first sensor system allowing for fetal ECG isolation; and/or processing the output signals of the first and second sensor systems to subtract motion artifact in physiological signal calculations for respiratory rate and/or heart rate.

In one embodiment, the method further comprises associating the wirelessly transmitted physiological parameters with a unique patient identifier, thereby identifying the physiological parameters with the subject in at least one of the patient database, the cloud server, and the mobile device.

In one embodiment, the method further comprises continuously monitoring one or more critical parameters associating at least one vital sign; and notifying a practitioner or caregiver when a sensor aberrant signal output condition occurs; and generating an alarm when an alarming vital sign reading condition in which the one or more of the critical parameters are out of pre-defined ranges occurs, and notifying a practitioner or caregiver of the alarm. In one embodiment, the one or more critical parameters are one or more of the heart parameters, temperature, body movements, respiratory parameters, oxygenation, and blood pressure.

In one embodiment, the method further comprises identifying a normal vital sign reading condition, an aberrant sensor output condition, or an alarming vital sign reading condition, based on the physiological parameters; and notifying a practitioner or caregiver when the sensor aberrant signal output condition occurs, and/or when the alarming vital sign reading condition in which the one or more of the critical parameters are out of pre-defined ranges occurs.

In one embodiment, the method further comprises assessing the physiological signs of the fetus and the mother during labor, based on the detected physiological parameters; assessing the physiological signs of the fetus and the mother for women with high-risk obstetrical complications prior to labor; and predicting for the onset of true labor from false labor.

In yet another aspect, the invention relates to a non-transitory tangible computer-readable medium storing instructions which, when executed by one or more processors, cause the above disclosed method to be performed.

### BRIEF DESCRIPTION OF THE DRAWINGS

The accompanying drawings illustrate one or more embodiments of the invention and together with the written description, serve to explain the principles of the invention. Wherever possible, the same reference numbers are used throughout the drawings to refer to the same or like elements of an embodiment.
FIGS. 1A-1H show schematically exploded, front-perspective and back-perspective views of different sensor units, and a deployment of the sensor units on a pregnant woman, according to certain embodiments of the invention. FIGS. 1A and 1D: an abdominal sensor unit, FIGS. 1B and 1E: a chest sensor unit, FIGS. 1C and 1F: a limb sensor unit, and FIGS. 1G and 1H: the deployment of the abdominal, chest and limb sensor units on a pregnant woman.
FIGS. 2A-2B show schematically a sensor network, according to certain embodiments of the invention. FIG. 2A: the sensor network, and FIG. 2B: a block diagram of each sensor unit of the sensor network where signals are time synced, which allows for better derivation of fetal HR but also other parameters of interest such as pulse arrival time and pulse transit time, a surrogate marker of blood pressure.
FIGS. 3A-3C show respectively a schematic diagram of abdominal, chest and limb sensor units, according to certain embodiments of the invention.
FIG. 4 shows a schematic representing flow and electronics design for a Doppler device from data collection to display on a smartphone, according to certain embodiments of the invention.

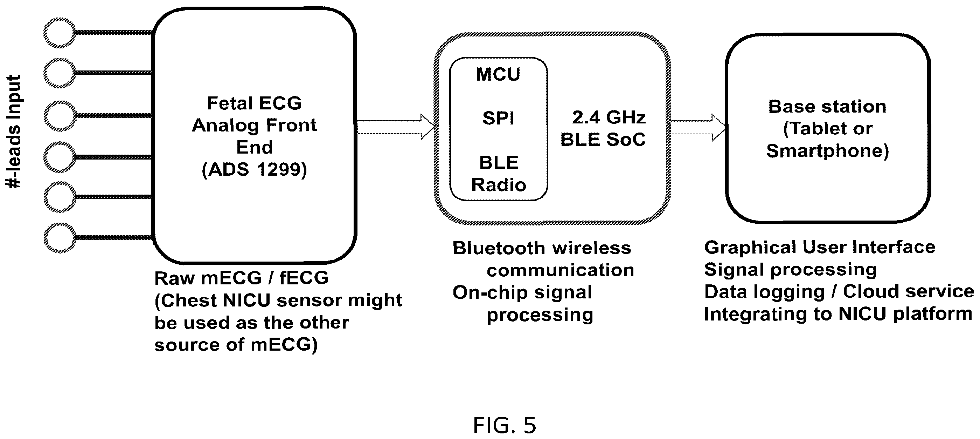

FIG. 5 shows schematically a fetal ECG unit with a number of potential leads to capture fetal ECG signals, according to certain embodiments of the invention. Analog front end includes ADS 1299 (Texas Instruments) with extreme low noise (1µ Vpp) and a high data rate (250 SPS to 16 kSPS).
FIG. 6 shows an 8 channel sensor specific to measuring fetal ECG including key components, according to certain embodiments of the invention. Key specifications: input referred noise, 1 µ Vpp; max sampling rate, 1 kHz (w/ internal clock); ADC resolution, 24 bit; amp gain (programmable), 1-24; max number of channels, 8; power consumption, 4 mW/channel; and overall footprint, 73 mm × 25 mm. PCB design: four islands, ECG AFE + signal pad, BLE + memory, power management + battery, and NFC coil.
FIG. 7 shows schematically a board layout of a fetal ECG sensor, according to certain embodiments of the invention.
FIG. 8 shows schematically a board diagram of a fetal ECG sensor with labels of key components, according to certain embodiments of the invention.
FIGS. 9A-9C show schematically mechanics of a fetal ECG sensor, according to certain embodiments of the invention. FIG. 7A: a load free state, FIG. 7B: a stretching state, and FIG. 7C: a twisting state.
FIG. 10 shows schematically a fetal ECG unit with one possible configurations of electrodes, according to certain embodiments of the invention. HUB is reusable and includes a low noise analog front end (AFE), a BLE communication module, a rechargeable battery (wired or wireless connection), and a PCB. Electrodes are disposable and include 5-lead with sufficient spacing for fetal HR recording and flexible interconnections between the electrodes and HUB.
FIGS. 11A-11B show schematically assembly of an abdominal sensor unit with an ultrasonic transducer, according to certain embodiments of the invention. After the 2-layer flexible printed circuit board is assembled with circuit components, sub-islands are folded.
FIG. 12 shows schematically a flowchart of fetal ECG signal processing through identifying and de-noising maternal ECG signals, according to certain embodiments of the invention.
FIG. 13 shows schematically a flowchart of the signal process in obtaining fetal heart rate from the Doppler unit, according to certain embodiments of the invention. Initial signal processing: band pass filtering (200-1500 Hz) for acoustic range, and envelope. Autocorrection approach: estimating periodically of Doppler signal, dynamic adjustment of autocorrection window, adaptive peak detection, and beat-to-beat heart rate determination.
FIG. 14 shows schematically a deployment of a sensor network system with multiple abdominal units, according to certain embodiments of the invention.
FIG. 15 shows schematically a user interface, according to certain embodiments of the invention.

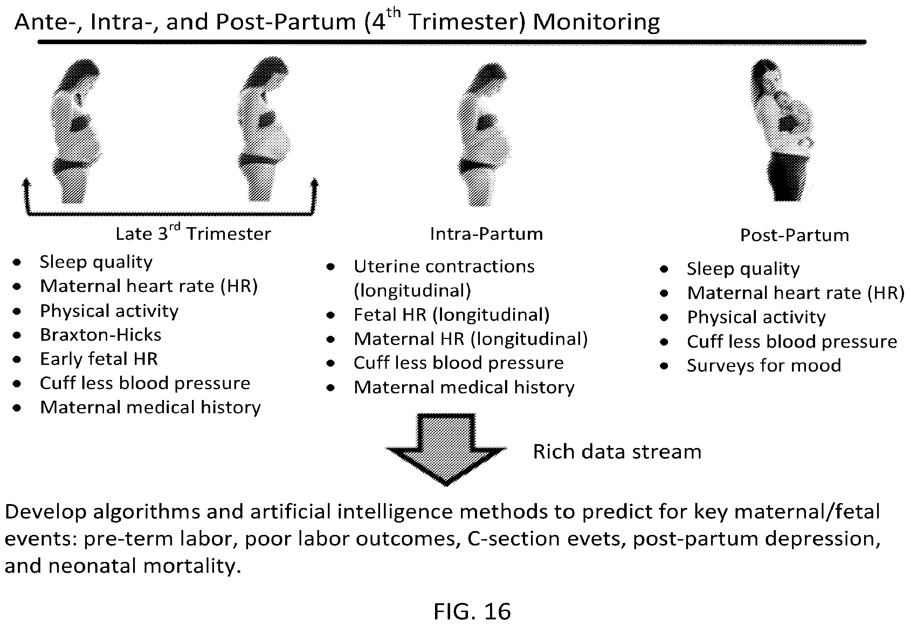

FIG. 16 shows schematically one use case of a sensor network system for ante-, intra- and post-partum monitoring, according to certain embodiments of the invention, which includes the ability to potentially predict for bad outcomes in pregnancy just as preterm labor or post-labor complications.

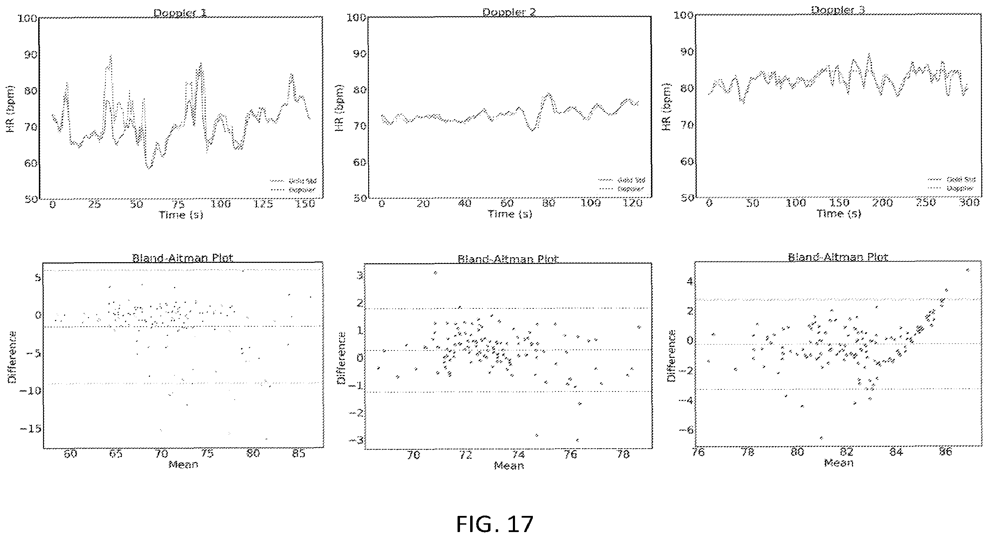

FIG. 17 shows the performance of the abdominal unit compared to a gold standard showing excellent agreement for Doppler derived fetal heart rate, according to certain embodiments of the invention.

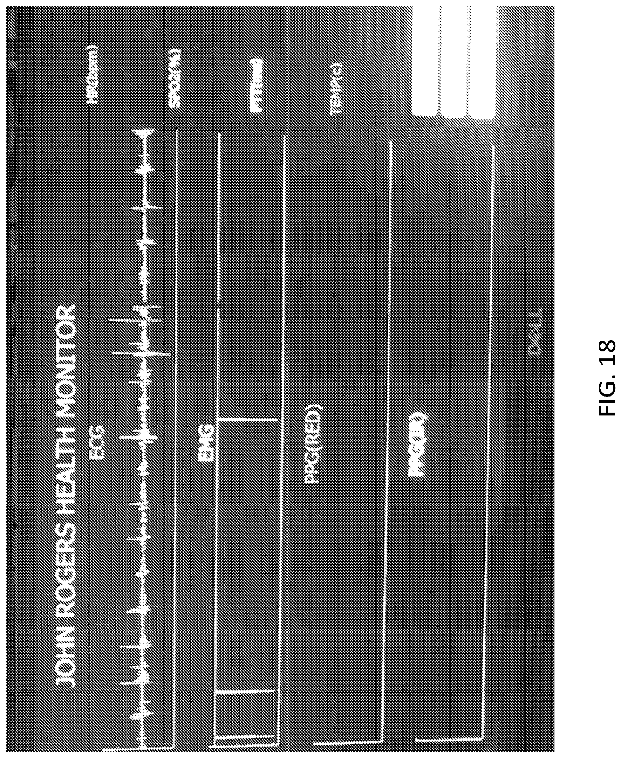

FIG. 18 shows a raw tracing of the Doppler output of the abdominal unit, according to certain embodiments of the invention.

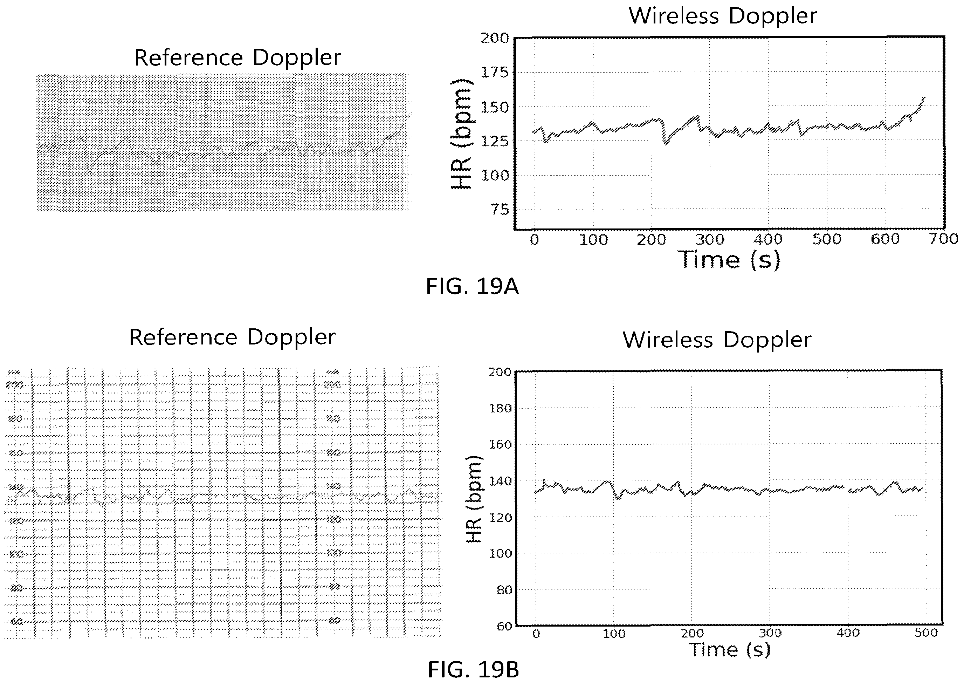

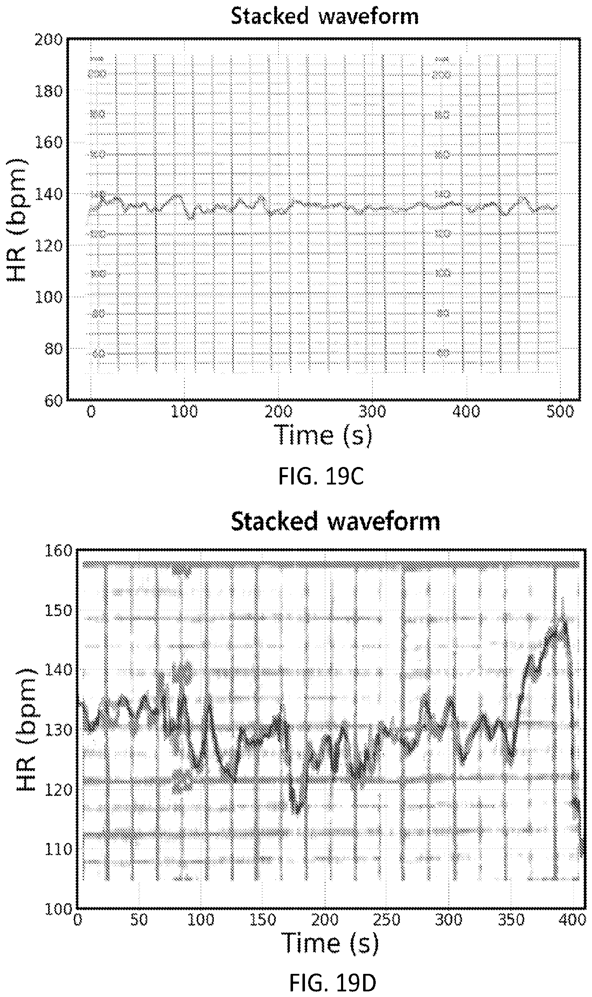

FIGS. 19A-19D show testing results of various subjects (all pregnant) with an abdominal unit for fetal heart rate, according to certain embodiments of the invention. FIG. 19A: for subject 1, FIGS. 19B-19C: for subject 2 with 34 weeks, and FIG. 19D: for subject 3 with 34 weeks.

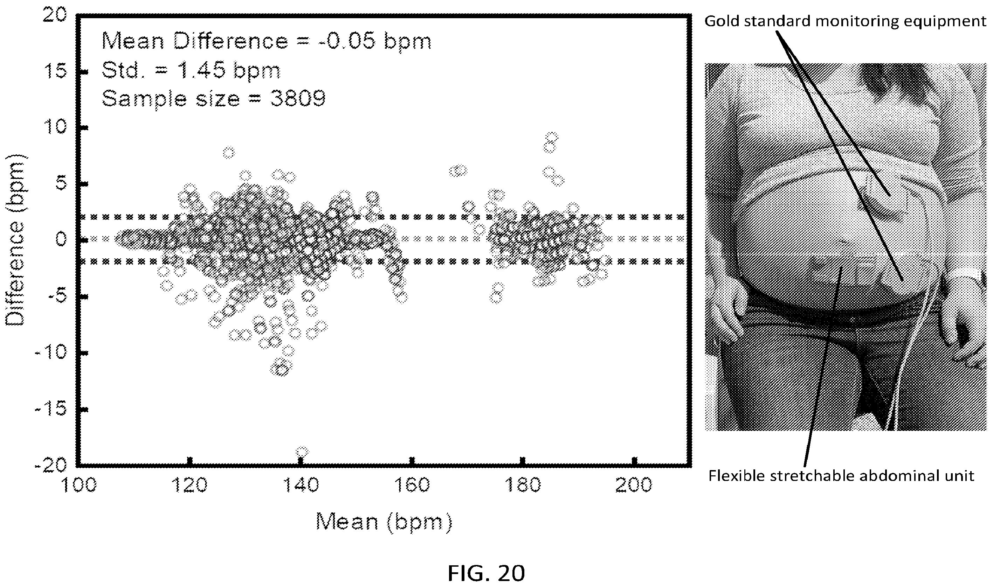

FIG. 20 illustrates summary data (left panel) on agreement for fetal heart rate between the flexible stretchable abdominal unit according to certain embodiments of the invention and gold standard monitoring equipment shown on the picture on the right panel.

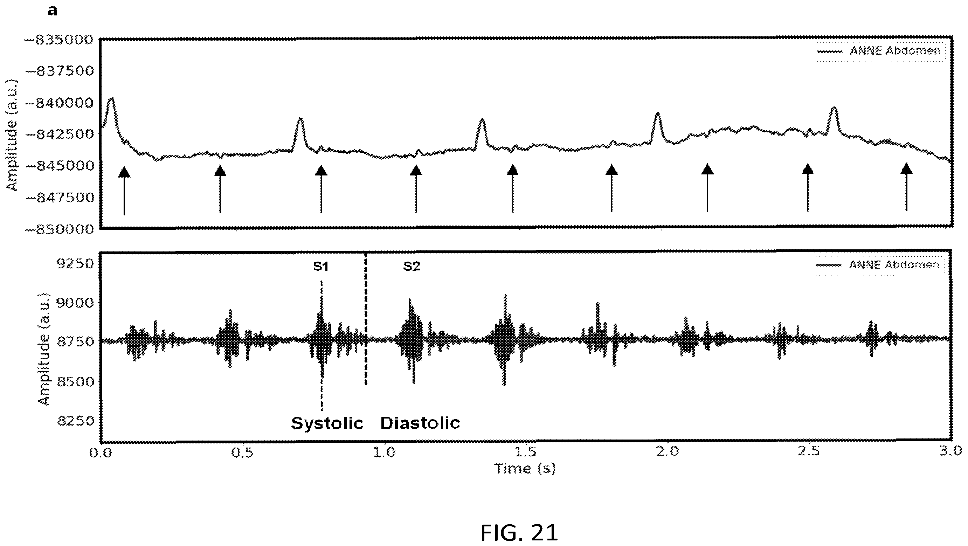

FIG. 21 shows longitudinal data indicative of fetal ECG outputs from abdominal/Doppler unit and Doppler signatures, according to certain embodiments of the invention.

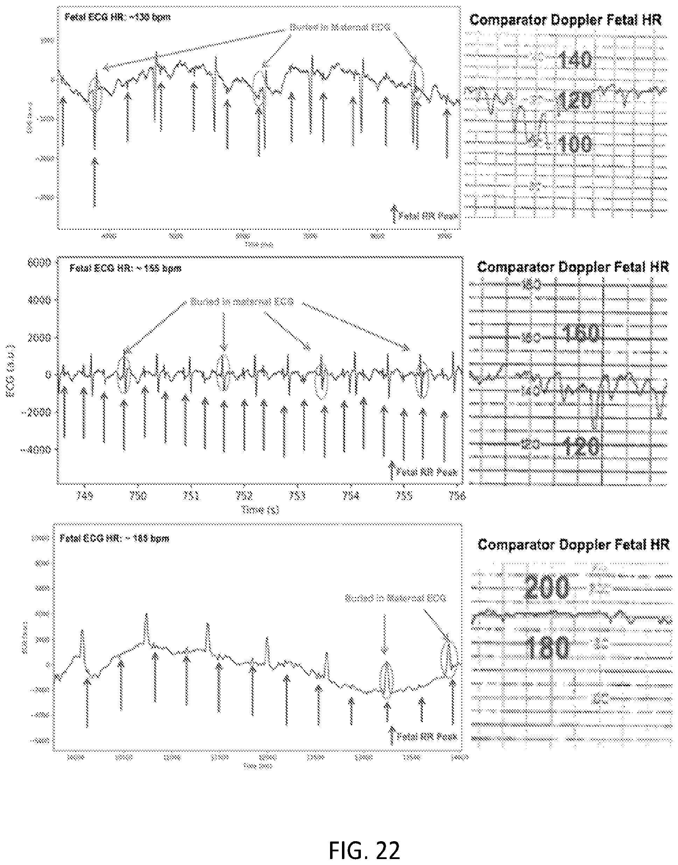

FIG. 22 shows fetal ECG traces collected from the abdominal sensor unit, according to certain embodiments of the invention.

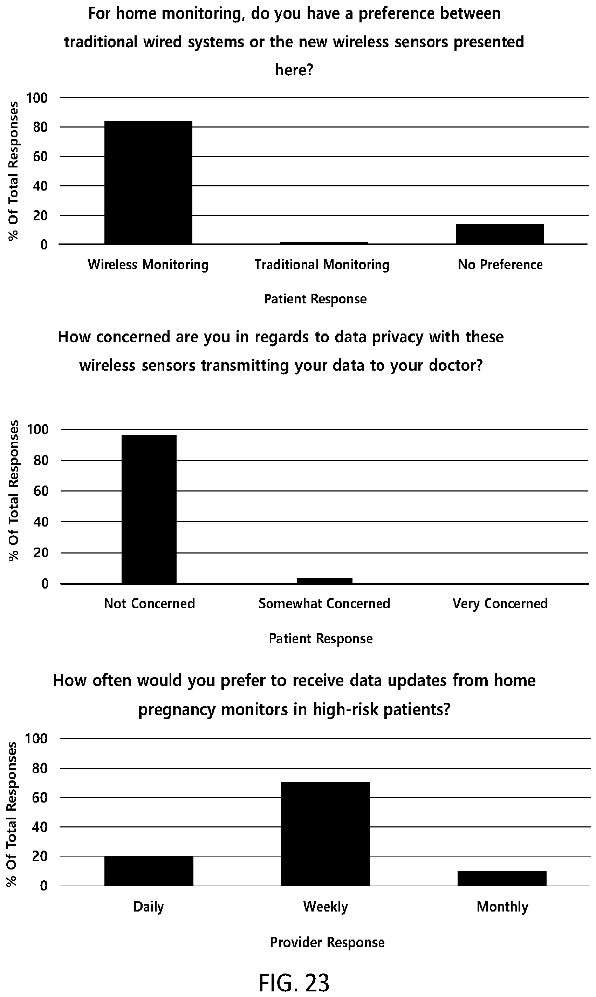

FIG. 23 shows patient reported preferences for wireless wearable sensors, according to certain embodiments of the invention.
FIGS. 24A-24E show various maternal vital signs detected with a sensor network, according to certain embodiments of the invention. The mean difference is -0.1 beats per minute for HR, -0.2% for SpO₂, and -0.5 breaths per minute for RR. The standard deviation is 1 beat per minute, 1.7% for SpO₂, and 1.6 breaths per minute for RR.
FIG. 25A shows schematically a flowchart of the signal process in obtaining the fetal heart rate from the Doppler unit, according to certain embodiments of the invention.
FIGS. 25B-25D show the fetal heart rate detected from the Doppler unit, according to certain embodiments of the invention.
FIGS. 26A-26C show various maternal vital signs detected with an abdominal sensor unit, according to certain embodiments of the invention.
FIGS. 27A-27B show blood pressures detected with the sensor network, according to certain embodiments of the invention.
FIGS. 28A-28D show time series of vital signs detected with the sensor network for laboring women, according to certain embodiments of the invention.
FIG. 29 shows various vital signs at different body position detected with the sensor network, according to certain embodiments of the invention.
FIGS. 30A-30B show feedback provided by a subset of pregnant women (FIG. 30A) and obstetrical nurses (FIG. 30B), respectively.

### DETAILED DESCRIPTION OF THE INVENTION

The invention will now be described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this invention will be thorough and complete, and will fully convey the scope of the invention to those skilled in the art. Like reference numerals refer to like elements throughout.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

One of ordinary skill in the art will appreciate that starting materials, biological materials, reagents, synthetic methods, purification methods, analytical methods, assay methods, and biological methods other than those specifically exemplified can be employed in the practice of the invention without resort to undue experimentation. All art-known functional equivalents, of any such materials and methods are intended to be included in this invention. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the invention has been specifically disclosed by preferred embodiments and optional features, modification and variation of the concepts herein disclosed may be resorted to by those skilled in the art, and that such modifications and variations are considered to be within the scope of this invention as defined by the appended claims.

Whenever a range is given in the specification, for example, a temperature range, a time range, or a composition or concentration range, all intermediate ranges and subranges, as well as all individual values included in the ranges given are intended to be included in the invention. It will be understood that any subranges or individual values in a range or subrange that are included in the description herein can be excluded from the claims herein.

It will be understood that, as used in the description herein and throughout the claims that follow, the meaning of "a", "an", and "the" includes plural reference unless the context clearly dictates otherwise. Thus, for example, reference to "a cell" includes a plurality of such cells and equivalents thereof known to those skilled in the art. As well, the terms "a" (or "an"), "one or more" and "at least one" can be used interchangeably herein. It is also to be noted that the terms "comprising", "including", and "having" can be used interchangeably.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the invention.

Furthermore, relative terms, such as "lower" or "bottom" and "upper" or "top," may be used herein to describe one element's relationship to another element as illustrated in the figures. It will be understood that relative terms are intended to encompass different orientations of the device in addition to the orientation depicted in the figures. For example, if the device in one of the figures is turned over, elements described as being on the "lower" side of other elements would then be oriented on "upper" sides of the other elements. The exemplary term "lower", can therefore, encompasses both an orientation of "lower" and "upper," depending of the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

It will be further understood that the terms "comprises" and/or "comprising", or "includes" and/or "including", or "has" and/or "having", or "carry" and/or "carrying", or "contain" and/or "containing", or "involve" and/or "involving", "characterized by", and the like are to be open-ended, i.e., to mean including but not limited to. When used in this disclosure, they specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the invention, and will not be interpreted in an idealized or overly formal sense unless expressly so defined herein.

As used in the disclosure, "around", "about", "approximately" or "substantially" shall generally mean within 20 percent, preferably within 10 percent, and more preferably within 5 percent of a given value or range. Numerical quantities given herein are approximate, meaning that the term "around", "about", "approximately" or "substantially" can be inferred if not expressly stated.

As used in the disclosure, the phrase "at least one of A, B, and C" should be construed to mean a logical (A or B or C), using a non-exclusive logical OR. As used herein, the term "and/or" includes any and all combinations of one or more of the associated listed items.

As used in the disclosure, the phrase "bidirectional wireless communication system" refers to onboard components of the sensor that provides capability of receiving and sending signals. In this manner, an output may be provided to an external device, including a cloud-based device, personal portable device, or a caregiver's computer system. Similarly, a command may be sent to the sensor, such as by an external controller, which may or may not correspond to the external device. Machine learning algorithms may be employed to improve signal analysis and, in turn, command signals sent to the medical sensor, including a stimulator of the medical sensor for providing haptic signal to a user of the medical device useful in a therapy. More generally, these systems may be incorporated into a processor, such as a microprocessor located on-board or physically remote from the electronic device of the medical sensor.

As used in the disclosure, the term "system-powered" refers to a configuration wherein a system or component thereof is capable of functioning or otherwise operating in a powered state for an operation period without relying on external power, such as without relying on external power provided by an external power source(s); e.g., wireless power coupled to the device via a power harvester. System-powered includes, for example, a system or component thereof that is powered via at least one "on-board" power source that is configured as a component of the system, such as via battery, fuel cell, solar cell, or the like provided as a part of the system. In an embodiment, the invention provides systems having one or more system-powered components that are powered via an on-board battery such as a button cell battery or coin cell battery. Incorporation of system-powered components in dosimetry systems of some embodiments allows for autonomous, ultra power efficient operation over useful operating periods, such as greater than 6 months, and in certain embodiments greater than 9 months and in some embodiments, greater than 1 year.

As used in this disclosure, the term "physical parameter" is used broadly herein, and may be one or more of heart rate, core body temperature, respiratory rate, peripheral temperature, blood oxygenation, blood pressure, uterine contraction frequency and intensity, fetal heart rate, and rates of change thereof.

As used in this disclosure, the term "data package" refers to transmission of information relevant to the measurement of the physical parameter, such as magnitude and time, and may be used for further calculations, including physical parameters and the like.

As used in this disclosure, the term "sensing" refers to detecting the presence, absence, amount, magnitude or intensity of a physical and/or chemical property. Useful device components for sensing include, but are not limited to, electrode elements, chemical or biological sensor elements, temperature sensors, strain sensors, mechanical sensors, position and orientation sensors, optical sensors, pulse oximeters, accelerometers and Doppler sensors.

As used in this disclosure, the term "encapsulate" refers to the orientation of one structure such that it is at least partially, and in some cases completely, surrounded by one or more other structures, such as a substrate, adhesive layer or encapsulating layer. "Partially encapsulated" refers to the orientation of one structure such that it is partially surrounded by one or more other structures, for example, wherein 30%, or optionally 50%, or optionally 90% of the external surface of the structure is surrounded by one or more structures. "Completely encapsulated" refers to the orientation of one structure such that it is completely surrounded by one or more other structures.

Embodiments of the invention are illustrated in detail hereinafter with reference to accompanying drawings. The description below is merely illustrative in nature and is in no way intended to limit the invention, its application, or uses. The broad teachings of the invention can be implemented in a variety of forms. Therefore, while this invention includes particular examples, the true scope of the invention should not be so limited since other modifications will become apparent upon a study of the drawings, the specification, and the following claims. For purposes of clarity, the same reference numbers will be used in the drawings to identify similar elements. It should be understood that one or more steps within a method may be executed in different order (or concurrently) without altering the principles of the invention.

Labor is the single most common reason for a hospital admission in the United States. While maternal mortality has drastically decreased in developed countries, giving birth remains one of the most dangerous things a woman will do in low and middle income countries (LMIC). Currently, monitoring devices and systems for labor are essential in the management of pregnant women before and during labor. However, existing technologies that measure key parameters associated with labor (fetal heart rate, maternal heart rate, maternal blood oxygenation, uterine contraction intensity / frequency, and maternal blood pressure require equipment that is teethered to large base units, bulky, and expensive. This represents an inconvenience in developed countries, while the absence of low cost monitoring systems for labor in LMICs directly contributes to increased maternal and neonatal morbidity and mortality.

To address the aforementioned deficiencies and inadequacies, the invention in one aspect discloses a sensor network system with Bluetooth enabled, battery-powered wearable patches for pregnancy monitoring of a woman throughout a wide range of gestational periods, without the need for wired systems. Bluetooth low energy (BLE) connectivity allows for the acquisition of data crucial for determining key instances throughout pregnancy in a compact, flexible form factor. The sensor network system can collectively measure maternal and fetal electrocardiograph (ECG), uterine electromyograph (EMG) (contraction intensity and frequency), maternal SpO₂, maternal skin temperature, and a surrogate marker for maternal blood pressure (pulse arrival time). Specifically, a novel wireless abdominal sensor is a maternal fetal unit designed with continuous/wearable Doppler function, fetal ECG, uterine EMG, and maternal ECG functionality. The abdominal sensor operates as a wireless body sensor network with other sensors to improve signal quality, specifically subtraction of maternal ECG from the abdominal sensor to isolate fetal ECG signals. This is the first wearable sensor system capable of recapitulating full vital signs monitoring for laboring women. The vital signs include, but are not limited to, physiological parameters such as a maternal heart rate, a respiratory rate (RR), a core body temperature, a peripheral temperature, a blood oxygenation, a blood pressure, uterine contraction frequency and intensity, and a fetal hear rate.

In certain embodiments, the sensor network includes a plurality of sensor systems time-synchronized to each other. Each sensor system is placed on a respective region of a pregnant woman and comprises a sensor member configured to detect data associated with at least one of physiological parameters of the subject, and a Bluetooth low energy system-on-a-chip (BLE SoC) configured to process and transmit the detected data. The sensor network also includes a controller or microcontroller unit (MCU) adapted in wireless communication with the plurality of sensor systems and configured to receive, from the plurality of sensor systems, to process, and to display the physiological parameters.

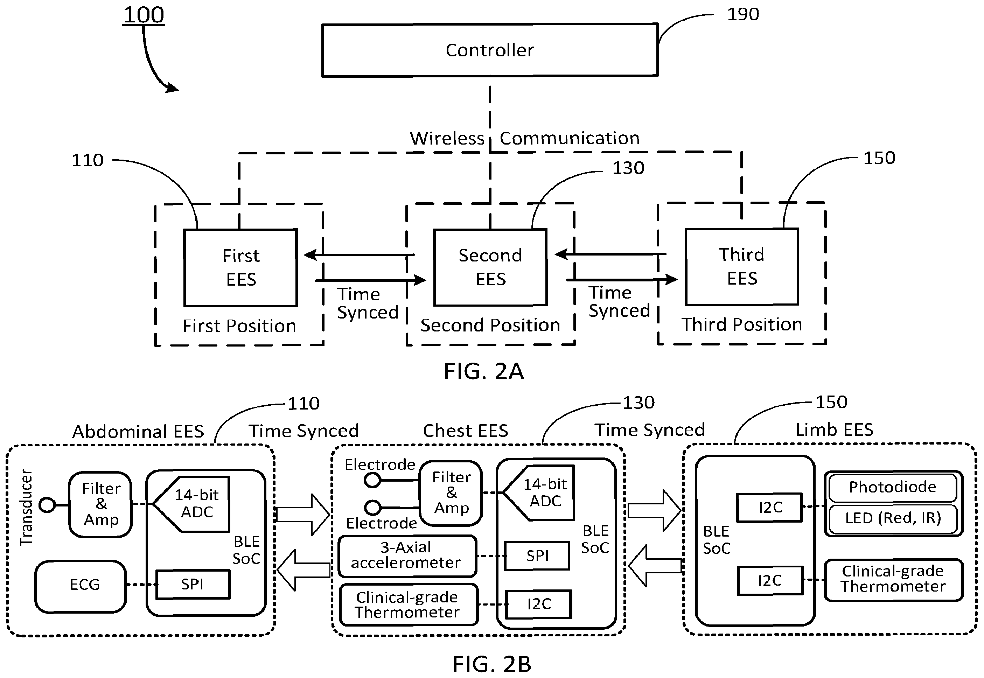

FIGS. 2A-2B schematically shows a functional block diagram of a sensor network according to one embodiment of the present invention. In the exemplary embodiment, the sensor network 100 includes first sensor system(s) 110, second sensor system(s) 130 and third sensor system(s) 150 that are time-synchronized to each other, and a controller (alternatively, MCU, or base station)190 adapted in wireless communication with the first sensor system 110 and the second sensor system 150. Specifically, the term "time-synchronized" (or "time-synced") refers to measurement of a parameter by different sensors, at different locations, that are synchronized in time to allow for measurement of novel physiological parameters. In some embodiments, the second sensor system 130 is configured to be a central node, while the first and third sensor systems 110 and 150 are configured to be peripheral nodes.

In certain embodiments, each of the first sensor system 110, the second sensor system 130 and the third sensor system 150 is in wireless communication with the MCU 190 via a wireless transmission protocol, such as Bluetooth protocol.

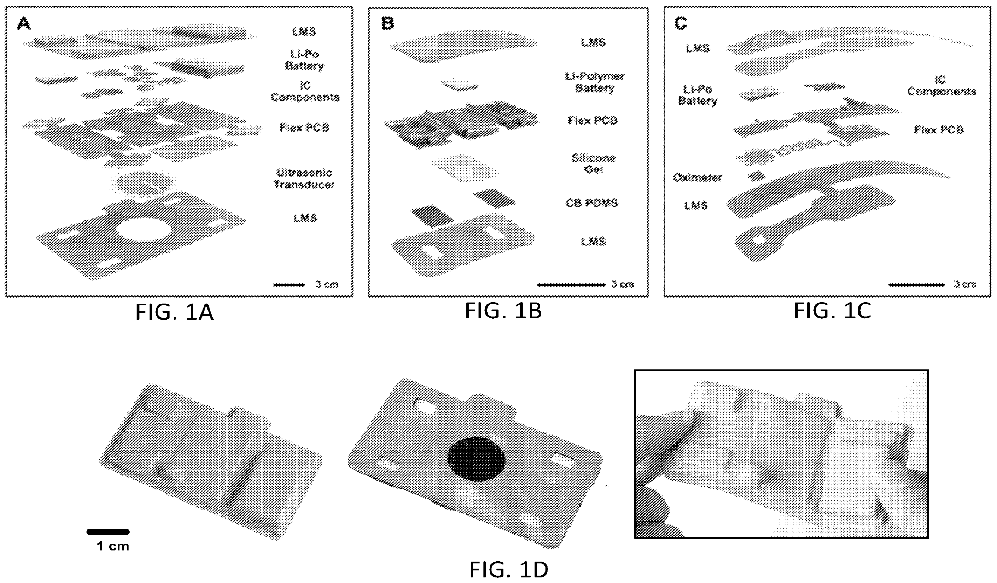

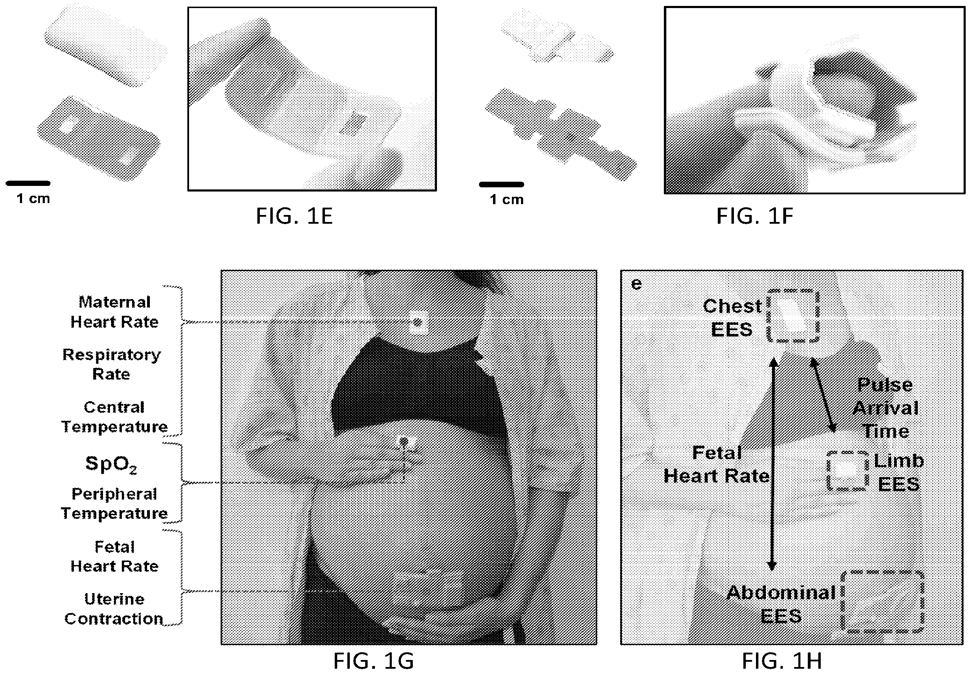

In certain embodiments, each of the first sensor system 110, the second sensor system 130 and the third sensor system 150 is an epidermal electronic system (EES), as shown in FIGS. 1A-1F. In certain embodiments as shown in FIGS. 1G-1H, the first sensor system 110 is a conformable chest abdominal system (alternatively, abdominal sensor unit or abdominal unit) configured to attach and conform to an abdominal region of a pregnant woman for recording the FHR, uterine contraction and/or the maternal heart rate; the second sensor system 130 is a conformable chest sensor system (alternatively, chest sensor unit or chest unit) configured to attach and conform to a chest region of the pregnant woman for recording a maternal heart rate, a core body temperature and/or a respiratory rate; and the third sensor system 150 is a conformable limb sensor system (alternatively, limb sensor unit or limb unit) configured to attach and conform to a limb region of the pregnant woman for recording photoplethysmogram (PPG) and/or a peripheral temperature, which are summarized in Table 1. In other words, the first sensor system 110 can be a Doppler, ECG/EMG sensor system, the second sensor system 110 can be an ECG sensor system, and the third sensor system 150 can be a PPG sensor system. Hereinafter, the first sensor system 110, the second sensor system 130 and the third sensor system 150 are also referred to an abdominal sensor unit (or abdominal unit), a chest sensor unit (or chest unit) and a limb sensor unit (or limb unit), respectively, in the disclosure.

**Table 1: Sensor Units and Physiological Parameters**

| | Physiological Parameters | Sensor Units |
|---|---|---|
| Maternal Vital Signs | Heart Rate | Wearable Chest Sensor Unit |
| | Core Body Temperature | • Electrocardiography (ECG) |
| | | • Inertial measurement unit (Accelerometer/Gyro) |
| | Respiratory Rate | • Skin temperature sensor |
| | Peripheral Temperature | Wearable Limb Sensor Unit |
| | Blood Oxygenation | • Pulse oximeter |
| | | • Skin temperature sensor |
| | Blood Pressure | PAT/PTT derived from Chest/Limb Sensor |
| Fetal Monitoring | Uterine Contraction Frequency / Intensity Fetal Heart Rate | Wearable Abdominal Sensor Unit |
| | | • Electromyography (EMG) |
| | | • Doppler |
| | | • Fetal ECG |

In certain embodiments, each of the abdominal sensor system 110, the chest sensor system 130 and the limb sensor system 150 includes a sensor member (circuit) having one or more sensors that are used to detect a vital sign of the pregnant woman, and then to generate one or more corresponding physiological parameters, a BLE SoC coupled to the sensor member (circuit) for receiving data from the sensor member and processing the received data, and wireless data transmission and wireless power harvesting. The sensors may be various types of sensors for detecting the vital sign as a signal, and the signal can be, for example, an electrical signal related to at least one of ECG and EMG technology; a mechanical signal related to movement, respiration and arterial tonometry; an acoustic signal related to fetal heart rate; and an optical signal related to blood oxygenation, and so on.

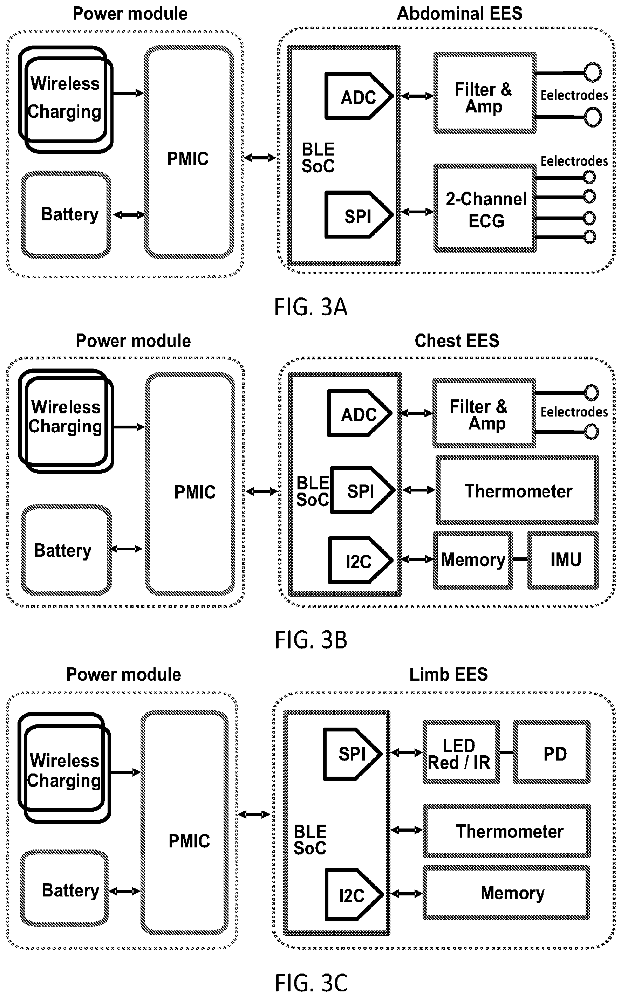

Referring to FIGS. 2B, 3A-3C, functional block diagrams of core components of the abdominal sensor system, the chest sensor system and the limb sensor system are shown according to embodiments of the invention, which include transducer (Doppler), analog-front-end for ECG/EMG processing and the BLE SoC for the abdominal EES; analog-front-end for ECG processing, 3-axis accelerometer, thermometer IC and the BLE SoC for the chest EES; and pulse oximeter, thermometer and the BLE SoC for the limb EES.

The abdominal EES includes two main modules: the Doppler module and the EMG module. The Doppler module includes an onboard wireless Doppler US device for continuously acoustic detection of the FHR. The Doppler module serves to measure fetal phonocardiography through the use of ultrasound waves. In one embodiment, a piezoelectric transducer is incorporated in the transmitter to resonate at 3MHz, which is received by a second transducer. In operation, standard ultrasound gel is used on the central component of the abdominal EES for fetal Doppler. The wireless abdominal EES operably generates an ultrasound waveform at a set frequency via the onboard transducer. The signal penetrates soft tissue and is reflected by abrupt changes in acoustic impedance. Upon reflection, the waves are altered to a different frequency with the Doppler Effect. The waves are then demodulated to the appropriate frequency for further signal processing. Through analog feedback and cascaded filtering one can obtain a fetal phonocardiogram at a high resolution to identify the S1 (closure of the tricuspid and mitral valves) and S2 (closure of the pulmonary semilunar valves) peaks. The algorithm filters the data through Hilbert transforms, autocorrelation, prominent peak detection, and segment shifting to produce robust fetal heart rate comparable to the standard GE Corometrics.

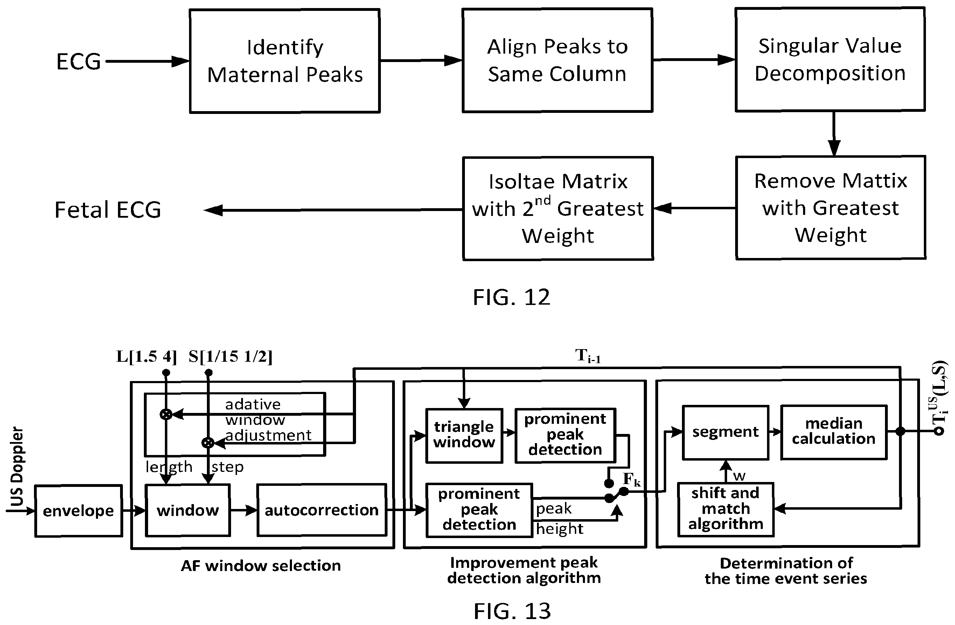

FIG. 13 shows schematically a flowchart of the signal process in obtaining the fetal heart rate via wireless ultrasound. Initially, the audible fetal phonocardiogram from the Doppler US is high pass filtered (200-1500 Hz) to obtain an envelope function. Autocorrection approach: estimating periodically of Doppler signal, dynamic adjustment of autocorrection window, adaptive peak detection, and beat-to-beat heart rate determination. First, the audible fetal phonocardiogram from the Doppler US is high pass filtered (f_{c} = 1 Hz), fed through a Hilbert transform algorithm and then scaled to obtain a positive envelope function. The envelope function is then segmented and processed with an autocorrelation function to identify peaks of recurring frequencies. The intervals of these frequencies are continuously updated and used to calculate FHR.

For the EMG module, a low-noise, 4-channel (expandable to 12) ADC is integrated for biopotential measurements. A total of four electrodes are placed at the extremities of the sensor - two for channel readings, one for reference, and one for bias measurement. The main functionality of this unit is to capture ECG from the body. With the sensor attached on the abdomen of a pregnant patient, it can capture the signals from both the mother and the fetus. The functionalities include, but are not limited to, fetal ECG, fetal heart rate, maternal ECG, maternal heart rate, uterine contraction, and lead-off detection. The EMG module is adapted for ultra-low noise operation for fetal ECG where multiple lead inputs (up to 12) can be used to interrogate for fetal HR, and women's health where maternal ECG (via a chest unit) may be subtracted from a maternal ECG / fetal ECG signal (via abdominal unit) to yield a clean fetal ECG signal (this reduces need for skin preparation / stripping; reduces number of electrodes). One embodiment of fetal ECG signal processing through identifying and de-noising maternal ECG signals is shown in FIG. 12.

The chest EES includes an ECG sensing unit, a motion sensing unit through a 3-axial accelerometer, and a clinical-grade thermometer. The ECG sensing unit includes two gold plated electrodes, an instrumentation amplifier, analog filters, and amplifiers incorporated in BLE SoC. Data acquisition of the motion sensing by the accelerometer is controlled by BLE SoC through serial peripheral interface (SPI) communication protocol, while the temperature data by the thermometer is acquired through the inter-integrated circuit (I²C) communication protocol, as shown in FIG. 2B Alternatively, the data acquisition of the motion sensing by the accelerometer is controlled by BLE SoC through the I²C communication protocol, while the temperature data by the thermometer is acquired through the SPI communication protocol, as shown in FIG. 3B The motion sensing unit may comprise an inertial measurement unit (IMU). The accelerometer or the IMU can be used with a motion artifact module to identify a vital sign as being subject to motion artifact and to correct of motion artifact.

The limb EES comprises a pulse oximetry for detecting blood oxygenation (SpO₂) and a thermometer for detecting the peripheral temperature. Both the pulse oximetry and thermometer are controlled by the BLE SoC through the I²C communication protocol, as shown in FIG. 2B. Alternatively, as shown in FIG. 3C, the SpO₂ and the peripheral temperature can be acquired by the pulse oximetry and the thermometer through the SPI and I²C SPI communication protocols, respectively.

As shown in FIGS. 3A-3C and 6-8, each of the abdominal EES, the chest ESS and the limb EES also includes a power module coupled to the BLE SoC for providing power to each EES. The power module comprises a power management integrated circuit (PMIC) coupled to the BLE SoC, and a battery coupled to the PMIC for providing power to each sensor system.

The battery is preferably a rechargeable battery. In this case, the power module further comprises a wireless charging module coupled to the PMIC for wirelessly charging the rechargeable battery. The wireless charging module in certain embodiments comprises a near-field communication (NFC) antenna.

In addition, the power module also includes a failure prevention element including a short-circuit protection component or a circuit to avoid battery malfunction.

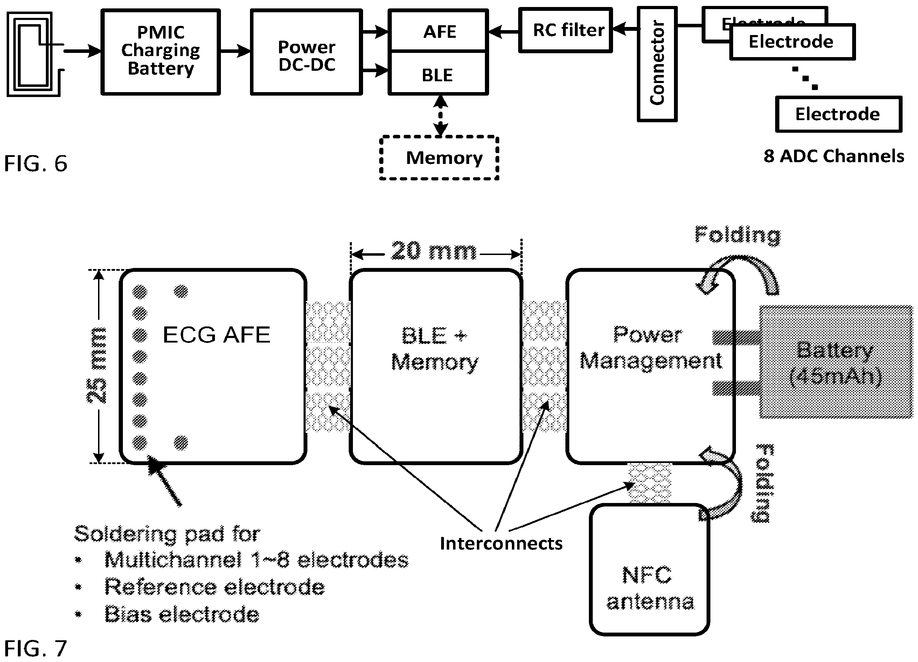

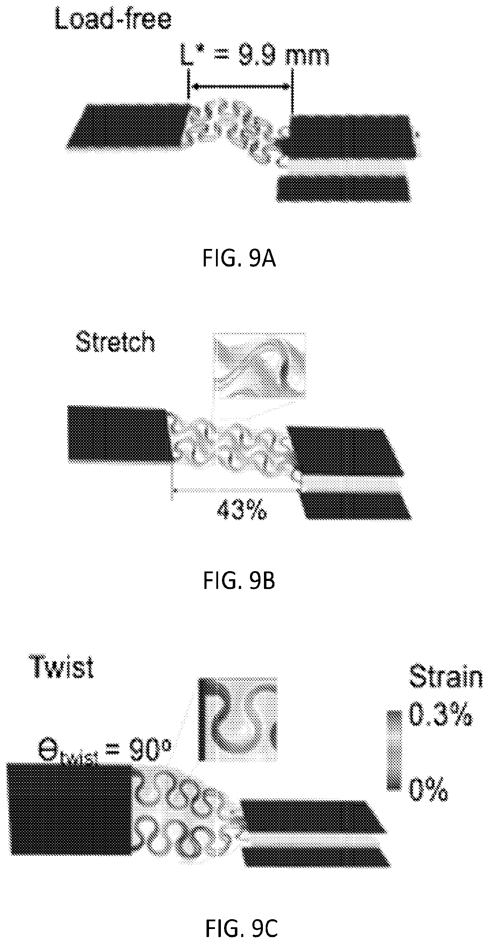

Furthermore, each of the abdominal EES. the chest ESS and the limb EES also includes a plurality of flexible and stretchable interconnects electrically connecting to EES' electronic components or ICs including the sensor member, the BLE SoC and the power module. FIGS. 7-10 shows schematically examples of flexible and stretchable interconnects and their connections to the sensor AFE, the BLE SoC and the power module for the abdominal EES.

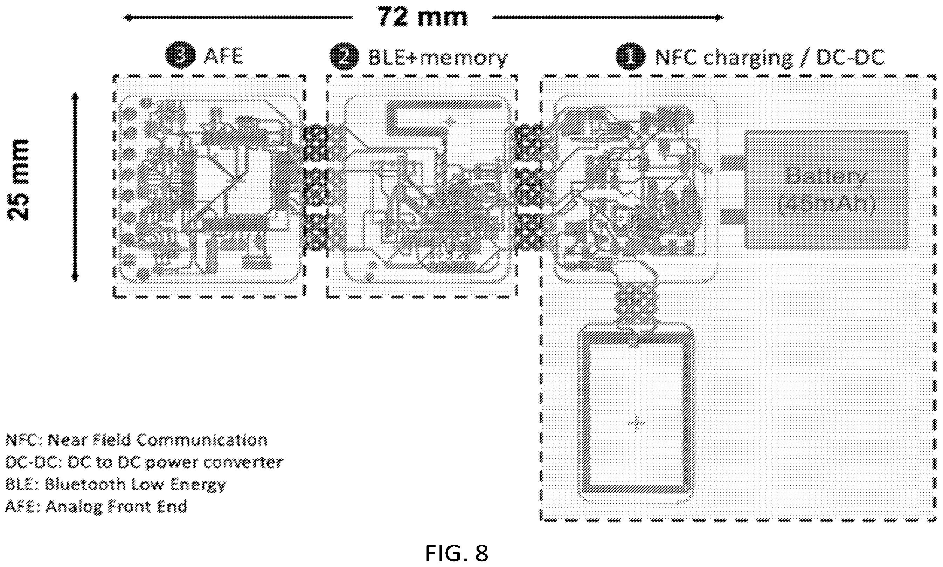

Moreover, each of the abdominal EES, the chest ESS and the limb EES further include one or more foldable electronic boards, where the electronic components and the flexible and stretchable interconnects are disposed on the one or more foldable electronic boards, as shown in FIGS. 8-9 and 11A-11B for example. As such, each EES is not only foldable to reduce its dimensions, but also conformable to the skin surface of the attached area/region of the pregnant woman.

As shown in FIGS. 1A-1F, 10 and 11A-11B, each of the abdominal EES, the chest ESS and the limb EES also includes an elastomeric encapsulation layer at least partially surrounding the electronic components and the flexible and stretchable interconnects to form a tissue-facing surface attached to the subject and an environment-facing surface. The tissue-facing surface is configured to conform to a skin surface of the pregnant woman. The encapsulation layer is formed of a flame retardant material. In one embodiment, the elastomeric encapsulation layer is a waterproof and biocompatible silicone enclosure.

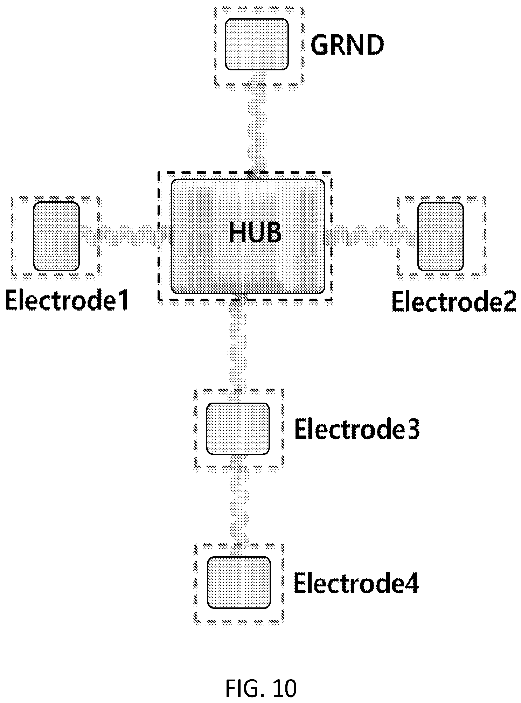

FIG. 10 shows schematically a fetal ECG unit with one configurations of electrodes, according to certain embodiments of the invention. HUB is reusable and includes a low noise analog front end (AFE), a BLE communication module, a rechargeable battery (wired or wireless connection), and a PCB. Electrodes are disposable and include 5-lead with sufficient spacing for fetal HR recording and flexible interconnections between the electrodes and HUB.

In addition, each of the abdominal EES and the chest EES further comprises a biocompatible hydrogel adhesive for attaching said EES on the respective region of the pregnant woman. The biocompatible hydrogel adhesive is adapted such that signals from the pregnant woman are operably conductible to said EES.

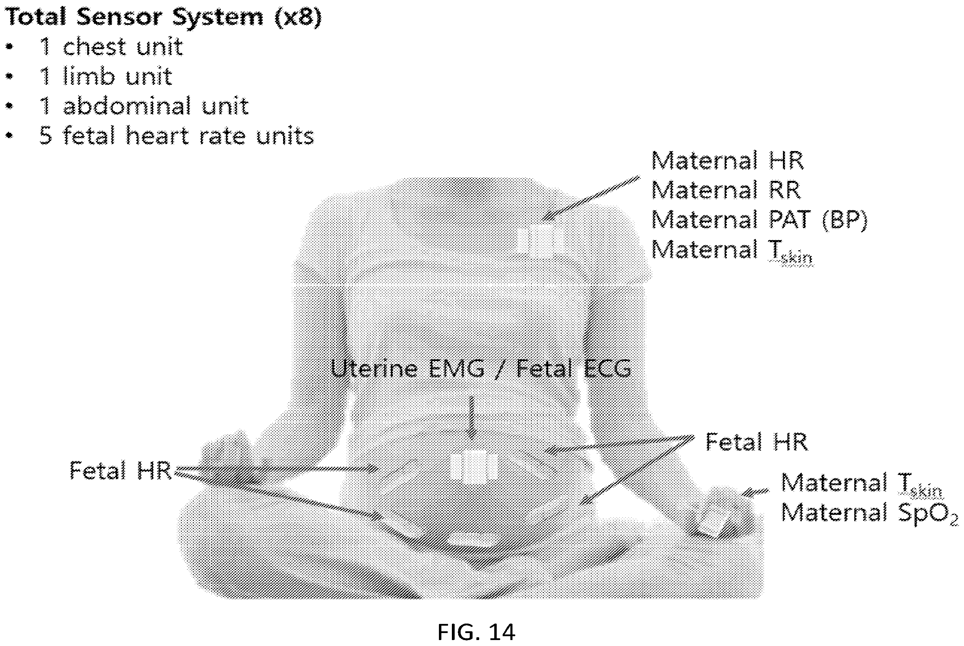

In one embodiment as shown in FIG. 14, a deployment of a sensor network system may include multiple abdominal units, e.g., one for uterine EMG and fetal ECG, five fetal ECG sensors for fetal HR detection.

In some embodiments, the controller comprises a mobile device operable serving as a base station for Bluetooth communication with the plurality of sensor systems including the abdominal EES, the chest ESS and the limb EES in the sensor network system. In one embodiment, the mobile device in bi-directionally wireless communication with the plurality of sensor systems.

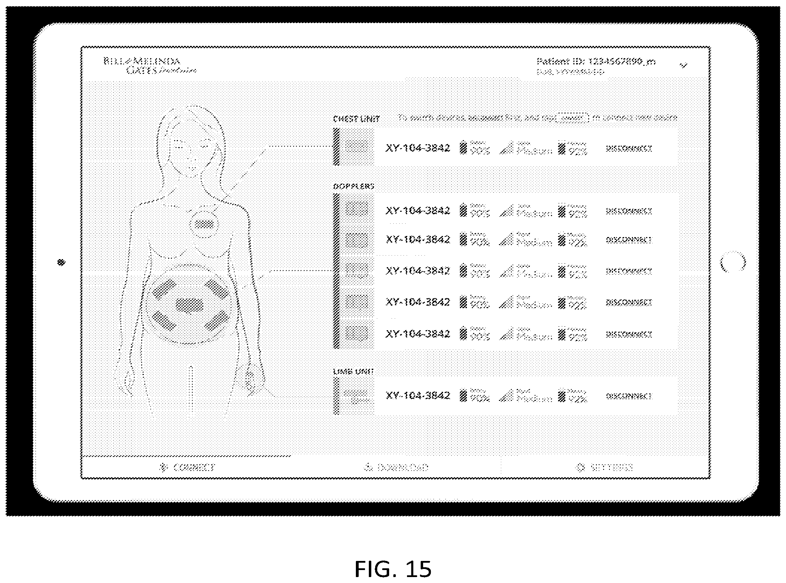

The mobile device can be a smartphone, a tablet, a hand-held device or a portable device having a graphical user interface to display the physiological parameters, as shown in FIG. 15.

In some embodiments, the controller is configured to perform at least one of the following functions: receiving and processing the detected data of the physiological parameters from the plurality of sensor systems; displaying the processed data of the physiological parameters in real time; transmitting the processed data of the physiological parameters to at least one of a patient database, a cloud server, and a mobile device; continuously monitoring one or more critical parameters associating at least one vital sign; and notifying a practitioner or caregiver when a sensor aberrant signal output condition occurs; and generating an alarm when an alarming vital sign reading condition in which the one or more critical parameters are out of pre-defined ranges occurs, and notifying a practitioner or caregiver of the alarm. The one or more critical parameters can be one or more of the heart parameters, temperature, body movements, respiratory parameters, oxygenation, and blood pressure.

In one embodiment, said processing the detected data of the physiological parameters comprises processing the output signals of the second and third sensor systems to determine a PAT and a PTT so as to obtain the blood pressure; eliminating the maternal ECG from the outputs of the first sensor system allowing for fetal ECG isolation; and/or processing the output signals of the first and second sensor systems to subtract motion artifact in physiological signal calculations for respiratory rate and/or heart rate. For instance, the sensor on the chest (accelerometer) can measure chest wall movement for respiratory rate and also heart rate with each beat. However, the performance deteriorates in instances where there is a lot of body motion (as in the case of pregnancy). In this instance, the accelerometer output from the abdominal sensor can be used to subtract from the accelerometer output from the chest sensor - isolating respiratory rate and heart rate even in high motion settings.

In one embodiment, the controller is configured to further perform the functions of assessing the physiological signs of the fetus and the mother during labor, based on the detected physiological parameters; assessing the physiological signs of the fetus and the mother for women with high-risk obstetrical complications prior to labor; and/or predicting for the onset of true labor from false labor.

According to the invention, the maternal fetal monitoring system with a network of wearable wireless sensors is able to capture: maternal respiratory rate, maternal heart rate, maternal core body position, maternal skin temperature, pulse oximetry, uterine contractions, and fetal heart rate. Specifically, the wearable patch system incorporates two key sensing elements: continuous Doppler and ECG/EMG. The wearable Doppler systems enables continuous acoustic measurement of fetal heart rate. The ECG unit enables simultaneous capture of fetal ECG and maternal ECG. The number of electrodes is adaptable and adjustable with up to 12 separate channels. Multiplexing several abdominal units allow for even more potential electrodes for fetal heart rate extraction. The EMG unit enables measurement of uterine contraction frequency and intensity. All of the above measurements are time-synced to each other allowing the assessment of uterine contractions and fetal heart rate in time synchrony.

In certain embodiments, the invention has the following advantages: unique isolation of electrical components in each EES enables operation in labor. Battery is current isolated, which enhances the electrical safety to the mother and fetus. Further, the battery has failure preventions including short circuit protection and battery explosion circuitry. Electrodes are able to be adjusted in spacing - allowing for accommodation of subjects of drastically different abdominal sizes. Low power operation enables greater than 24 hours of continuous use between charges. Flame retardation of the elastomer encapsulation reduces risk of injury.

These sensors fully support Bluetooth technology, thereby enabling drastically extended sensing ranges with a base unit. Master-slave linked sensor system allows for time synced measurements of novel physiological parameters across the entire network of sensors.

Proprietary algorithms enables the determination of fetal heart rate from both fetal ECG and fetal Doppler measurements.

Dual heart rate sensing and processing for maternal and fetal heart rate include subtraction of maternal heart rate from the abdominal unit.

Dual measurement systems to capture accurate respiratory rate. The measurement of respiratory rate derived through the ECG via impedance pneumonography is often times inaccurate and overestimates the true respiratory rate. In one embodiment, the maternal fetal monitoring system has the ability to measure chest wall movements to derive respiratory rate along with traditional impedance pneumonography.

The signal processing algorithm of the fetal heart rate from the wireless Doppler leverages a Hilbert transform to trace the analytic envelope and uses autocorrelation periodic part is captured.

High frequency accelerometer enables an electrode-free method that negates the need for additional skin preparation for the measurement of the heart rate and the respiratory rate; signal processing enables distinction between chest wall movements associated with breathing compared to heart rate.

High frequency accelerometer enables assessment of a women's body position during labor - further analytics enable determination of body position and its effect on fetal heart rate with the goal of guiding.

In addition, advanced software function and interoperability enabling cloud storage, remote login, and secure communication. Further capabilities include the integration of third-party sensors, wearables, and other hardware systems. Paired integration with electronic health records.

The maternal fetal monitoring system is also adapted for assessing the physiological signs of the fetus and the mother during labor; assessing the physiological signs of the fetus and the mother for women with high-risk obstetrical complications (e.g. pre-eclampsia, palcenta previa) prior to labor; and/or predicting for the onset of true labor from false labor.

Broader applications from this new sensor system extends beyond labor to other areas of medical monitoring from critical care to ambulatory monitoring. For example, the system can be utilized in maternal fetal monitoring that enables time-synced measurement of uterine contractions, fetal heart rate, and maternal heart rate; in cardiology for inpatient or remote monitoring; specifically value in non-invasive quantification of cardiac output, stroke volume, and left ventricular ejection fraction via the onboard Doppler and Seismocardiogram; and/or in critical care settings where assessment of cardiac acoustics is of additional value.

Another aspect of the invention also discloses a method for pregnancy monitoring of a woman. The method comprises the following steps.

At first, a sensor network comprising a plurality of sensor systems is deployed on the pregnant woman. Each sensor system comprises a sensor member configured to detect a vital sign of the subject and generate a corresponding one of the physiological parameters. In some embodiments. The plurality of sensor systems comprises a first sensor system, a second sensor system and a third sensor system respectively placed on an abdominal region, a chest region and a limb region of the pregnant woman. The limb region can be a finger, toe, foot, wrist, forehead, or ear. In one embodiment, the first sensor system is an abdominal EES configured to detect the FHR, the uterine contraction, and/or the maternal heart rate, the second sensor system is a chest EES configured to detect the maternal heart rate, the core body temperature, and/or the RR, and the third sensor system is a limb EES configured to detect the PPG and/or the peripheral temperature.

The plurality of sensor systems is synchronized to a common time base.

After time-synchronized, data of the physiological parameters are detected from the plurality of sensor systems.

Next, the detected data of the physiological parameters from the plurality of sensor systems are processed. In one embodiment, said processing the detected data of the physiological parameters comprises processing the output signals of the second and third sensor systems to determine a PAT and a PTT so as to obtain the blood pressure; and/or eliminating the maternal ECG from the outputs of the first sensor system allowing for fetal ECG isolation; and/or processing the output signals of the first and second sensor systems to subtract motion artifact in physiological signal calculations for respiratory rate and/or heart rate. For instance, the sensor on the chest (accelerometer) can measure chest wall movement for respiratory rate and also heart rate with each beat. However, the performance deteriorates in instances where there is a lot of body motion (as in the case of pregnancy). In this instance the accelerometer output from the abdominal sensor can be used to subtract from the accelerometer output from the chest sensor - isolating respiratory rate and heart rate even in high motion settings.

The processed data of the physiological parameters are displayed in real time. Meanwhile, the processed data of the physiological parameters can be transmitted to at least one of a patient database, a cloud server, and a mobile device.

The method may further comprise associating the wirelessly transmitted physiological parameters with a unique patient identifier, thereby identifying the physiological parameters with the subject in at least one of the patient database, the cloud server, and the mobile device.

In addition, the method also comprises continuously monitoring one or more critical parameters associating at least one vital sign; and notifying a practitioner or caregiver when a sensor aberrant signal output condition occurs; and generating an alarm when an alarming vital sign reading condition in which the one or more of the critical parameters are out of pre-defined ranges occurs, and notifying a practitioner or caregiver of the alarm. In one embodiment, the one or more critical parameters are one or more of the heart parameters, temperature, body movements, respiratory parameters, oxygenation, and blood pressure.

Furthermore, the method comprises identifying a normal vital sign reading condition, an aberrant sensor output condition, or an alarming vital sign reading condition, based on the physiological parameters; and notifying a practitioner or caregiver when the sensor aberrant signal output condition occurs, and/or when the alarming vital sign reading condition in which the one or more of the critical parameters are out of pre-defined ranges occurs.

Moreover, the method also comprises assessing the physiological signs of the fetus and the mother during labor, based on the detected physiological parameters; assessing the physiological signs of the fetus and the mother for women with high-risk obstetrical complications prior to labor; and predicting for the onset of true labor from false labor.

It should be noted that all or a part of the methods according to the embodiments of the invention is implemented by hardware or a program instructing relevant hardware.

Yet another aspect of the invention provides a non-transitory computer readable storage medium/memory which stores computer executable instructions or program codes. The computer executable instructions or program codes enable a computer or a similar computing apparatus to complete various operations in the above disclosed methods for pregnancy monitoring of a woman. The storage medium/memory may include, but is not limited to, highspeed random access medium/memory such as DRAM, SRAM, DDR RAM or other random access solid state memory devices, and non-volatile memory such as one or more magnetic disk storage devices, optical disk storage devices, flash memory devices, or other non-volatile solid state storage devices.

These and other aspects of the present invention are further described in the following section. Without intending to limit the scope of the invention, further exemplary implementations of the present invention according to the embodiments of the present invention are given below. Note that titles or subtitles may be used in the examples for the convenience of a reader, which in no way should limit the scope of the invention. Moreover, certain theories are proposed and disclosed herein; however, in no way should they, whether they are right or wrong, limit the scope of the invention so long as the invention is practiced according to the invention without regard for any particular theory or scheme of action.

### EXAMPLE

### COMPREHENSIVE MATERNAL FETAL MONITORING WITH A NETWORK OF WIRELESS, SOFT AND FLEXIBLE SENSORS IN HIGH AND LOW RESOURCE HEALTH SETTINGS

Vital signs monitoring is a fundamental component of promoting health and safety of mothers and newborns during pregnancy, labor, and childbirth. It is the first step that allows for early detection of pregnancy abnormalities, providing an opportunity for prompt, effective intervention to address preventable causes of maternal morbidity and mortality. Conventional pregnancy monitoring systems requires a multitude of devices wired to large base units to assess maternal and fetal health status. However, more than five separate devices with distinct user interfaces are commonly used to independently detect fetal heart rate, uterine contractility, maternal oxygenation, temperature, heart rate, and blood pressure. The piecemeal systems of monitoring technologies are expensive and complex with implementation challenges in low resource settings where maternal morbidity and mortality is the greatest.

This exemplary example presents an integrated monitoring platform/system leveraging advanced flexible electronics, wireless connectivity, and compatibility with a wide range of low cost mobile devices. Specifically, the platform/system includes a network of soft, flexible, and wireless sensors that provide continuous, comprehensive, and non-invasive monitoring of both the mother and fetus applicable across the entire continuum of obstetrics compatible with a wide range of mobile phones, which offers advanced features such as continuous measurements of blood pressure, uterine electromyography, and automated body position classification. In one embodiment, the network of soft, flexible, and wireless sensors includes three flexible, soft, and low profile sensors offer comprehensive vital signs monitoring for both mother and fetus with time synchronized operation allowing for advanced parameters such as automated body position classification, continuous cuff-less blood pressure, and EMG-derived uterine monitoring. The data streams generated by this system are integrated with the cloud allowing for future machine-learning opportunities. Successful field trials in both high resource (Chicago, IL, USA) and low resource settings (Lusaka Zambia) for pregnant women (n=572) between 25 and 41 weeks gestation demonstrate the system's performance, usability, and safety.

### Wireless Maternal-Fetal Sensor System Design

FIGS. 1A-1E provide exploded, front and back views of the abdominal, chest, and limb sensors, respectively. The chest sensor (FIGS. 1B and 1E) is placed on the chest of a pregnant women (FIGS. 1G-1H) and operably measures electrocardiography (ECG), seismocardiogram (SCG), chest wall movement for respiratory rate (RR), and skin temperature, each sampled at 504, 100, 200, and 0.2 Hz, respectively. As shown in FIG. 2D, the chest sensor includes a highfrequency, 3-axial accelerometer (BMI160, Bosch Sensortec), clinical-grade thermometer (MAX30205, Maxim Integrated), and an ECG-sensing unit including two gold-plated electrodes, an instrumentation amplifier, analog filters, amplifiers, and an internal analog-to-digital converter (ADC) in the Bluetooth low energy (BLE) system-on-a-chip (SoC) (nRF52832, Nordic Semiconductor). The BLE SoC controls the motion-sensing unit through a serial peripheral interface (SPI) communication protocol and the thermometer through the inter-integrated circuit (I²C) communication protocol. Construction of the chest sensor utilizes folding of the sub-islands (FIG. 1B) for optimal distribution of IC components thereby reducing its two-dimensional surface area by about 250%. Pre-compressed serpentines provide elastic stretchability while inner layers of a low modulus soft material (Ecoflex^{™} Gel) to enhance flexibility and conformability.

The limb sensor, as shown in FIGS. 1C and 1F, operably measures photoplethysmogram (PPG) and skin temperature, sampled at 100 and 0.2 Hz, respectively. The limb sensor wraps around the index finger of the pregnant women (FIGS. 1G-1H) to collect photoplethysmogram (PPG) and peripheral skin temperature. As shown in FIG. 2B, the limb sensor includes an integrated pulse oximetry module (MAX30101, Maxim Integrated) to measure blood oxygenation (SpO₂) and a thermometer (MAX30205, Maxim Integrated) to measure skin temperature.

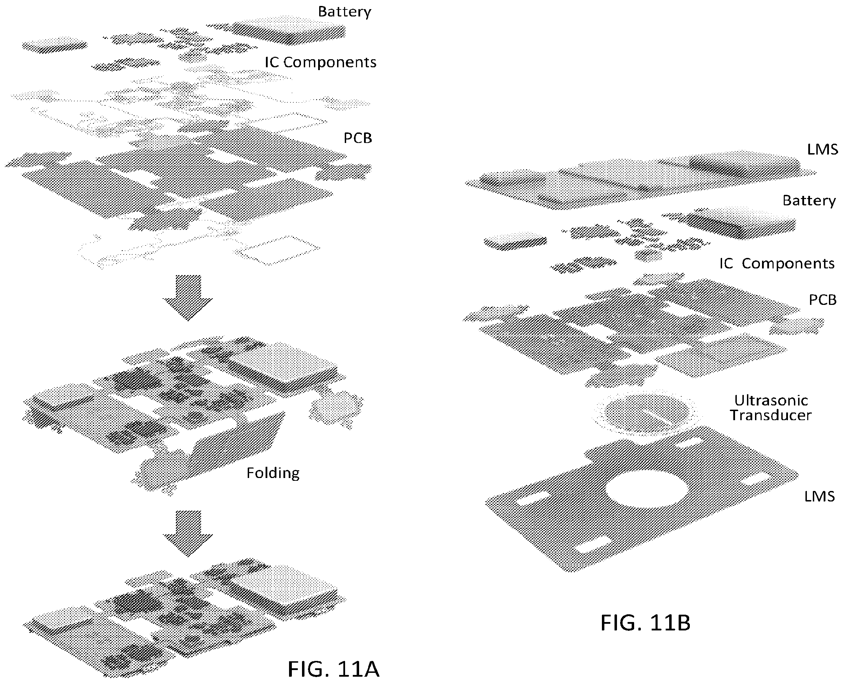

The abdominal sensor, as shown in FIGS. 1A and 1D, offers an onboard wireless Doppler ultrasound (US), and electromyograph (EMG) and electrocardiograph (ECG), each sampled at 504 and 500 Hz, respectively. Each sensor is composed of copper traces connecting the integrated circuit (IC) components and is fully encapsulated in a waterproof and biocompatible silicone enclosure. The abdominal sensor is operably placed on the pregnant woman's abdomen (FIGS. 1G-1H) to derive a fetal heart rate (FHR) via ultrasound, EMG to derive uterine contraction, ECG to derive a maternal heart rate. Construction of the abdominal sensor involves folding of the exterior planes (FIG. 11A-11B) for optimal distribution of IC components and reducing its two-dimensional surface area by about 24%. A piezoelectric transducer tuned to resonate at 3 MHz is incorporated as the transmitter and receiver for the Doppler Effect. Through analog feedback and cascaded filtering, the signal is fed into the BLE SoC. The EMG and ECG functionalities are generated by a low-noise, 4-channel ADC for biopotential measurements (ADS1299-4, Texas Instruments). A total of four electrodes are located on the lateral aspects of the abdominal sensors - two for channel readings, one for reference, and one for bias measurement. The BLE SoC controls the ICs through the I²C protocol.

FIGS. 2A-2B demonstrate the maternal-fetal monitoring system setup according to one one embodiment of the invention. All three sensors connect to a central mobile tablet (i.e., controller) that serves as the base station for Bluetooth communication and real-time data display. A biocompatible hydrogel adhesive conducts the signal from the patient to the chest and abdominal sensors. A fabric strap holds the limb sensor in place around the curvilinear index finger. Standard ultrasound gel is used on the central component of the abdominal sensor for fetal Doppler. FIGS. 1G-1H demonstrate the on-body network achieved via out of band time-synchronization of the three sensors with 1 ms timing accuracy. The chest unit acts as the central node with the abdominal and limb unit sensors acting as peripherals. The outputs of the chest and limb sensors are then capable of calculating both pulse arrival time (PAT) and pulse transit time (PTT), which serves as a surrogate measurement for blood pressure. The chest and abdominal sensors also communicate which enable maternal ECG elimination from the abdominal sensor's outputs allowing for the potential of future fetal ECG isolation. Real time comparison of the maternal heart rate and the fetal heart rate is also displayed on the software interface. FIGS. 2B and 3A-3C outline block diagrams illustrating the high-level architecture of the maternal-fetal monitoring system for the chest sensor, the limb sensor, and the abdominal sensor, according to one embodiment of the invention.

### Real-Time Measurement of Clinical Data for Maternal Health

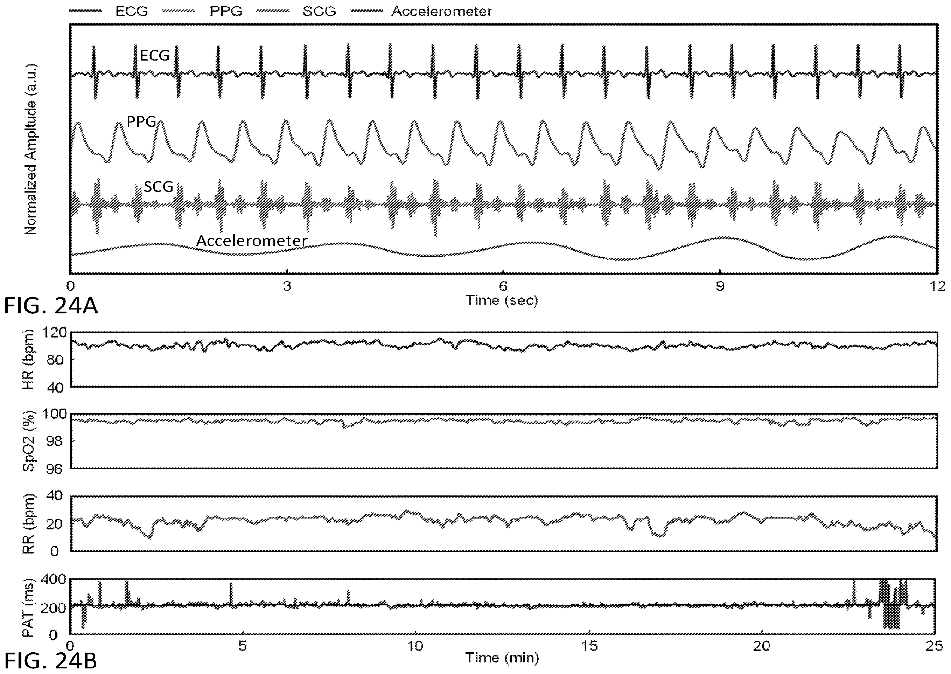

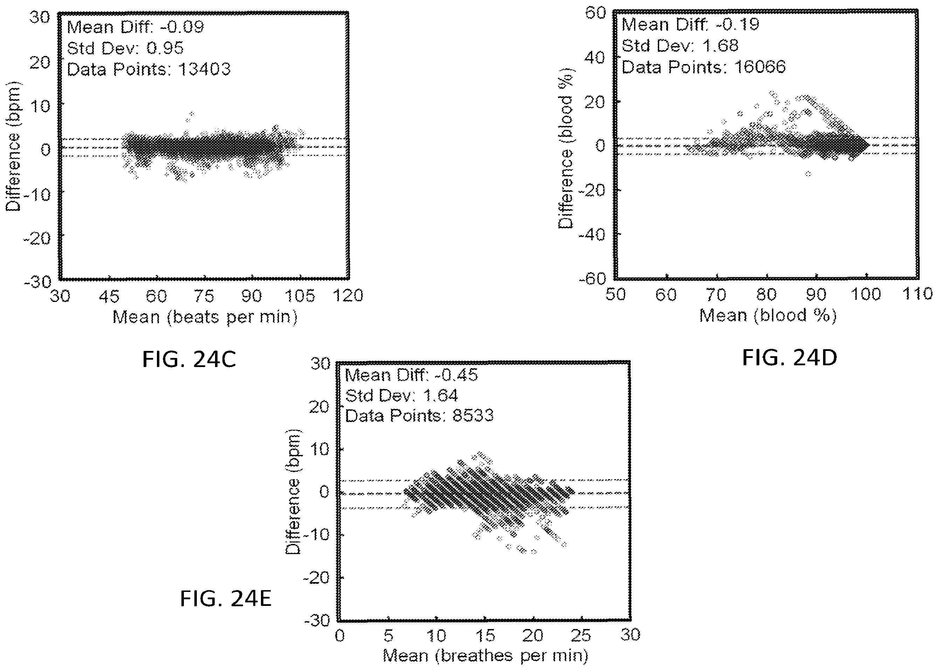

FIG. 24A summarizes the core sensor outputs of the wireless sensor network for maternal ECG, PPG, SCG, and z-axis accelerometry. The real-time data is processed via algorithms implemented directly on a mobile device to calculate vital signs. FIG. 24B depicts vital signs processed in real time from the sensor system in a time series format. A modified Pan-Tompkins algorithm filters the data and R-peak detection yields maternal heart rate (HR). Calculation of peripheral capillary oxygen saturation (SpO₂) involves a bandpass filter (f_{c1} = 0.5 Hz, f_{c2} = 5 Hz). Normalized PPG signals are processed with continuous wavelet transform (CWT), which constructs continuous time-frequency analysis of the signal. CWT detects rapid changes in frequency in the time domain potentially caused by motion-driven artifacts. An additional discrete saturation transform (DST) further removes motion artifacts through adaptive filtering, which cancels noise content based on a predetermined reference noise signal. Respiratory rate (RR) is captured by chest wall movement data obtained from the z-axis of the accelerometer. The data is bandpass filtered (f_{c1} = 0.1 Hz, f_{c2} = 1 Hz) and automated via a multiscale-based peak detection (AMPD) process. The 3-axis accelerometer outputs also enables real time classification of body position relevant to pregnancy based on the x, y, and z-axis baselines. Finally, continuous temperature on the chest and limb sensors allows for fever curve tracking. FIGS. 24C-24E demonstrate the agreement in heart rate, blood oxygenation, and respiratory rate calculations between the experimental system and a clinical gold standard monitoring system (Intellivue MP50, Philips) operating concomitantly in a cohort of n=13 healthy adults. The Bland-Altman method provides a quantitative comparison method and further supports similarity between the sensors and the gold standard. The mean difference is -0.09 beats per minute for HR, -0.19 % for SpO₂, and -0.45 breaths per minute for RR. The standard deviation is 0.95 beats per minute, 1.68 % for SpO₂, and 1.64 breaths per minute for RR. The differences are negligible and well within FDA guidelines for new monitoring platforms. Data can be streamed continuously or hidden for clinical research purposes using a wide range of mobile devices.

### Fetal Heart Rate and Uterine Contraction via a Soft, Flexible, and Wireless Abdominal Sensor

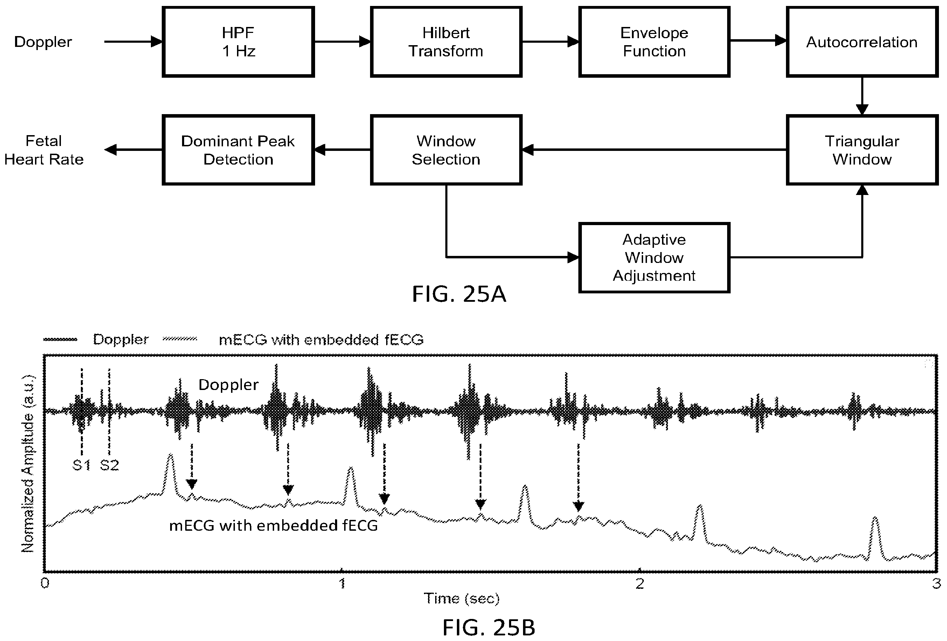

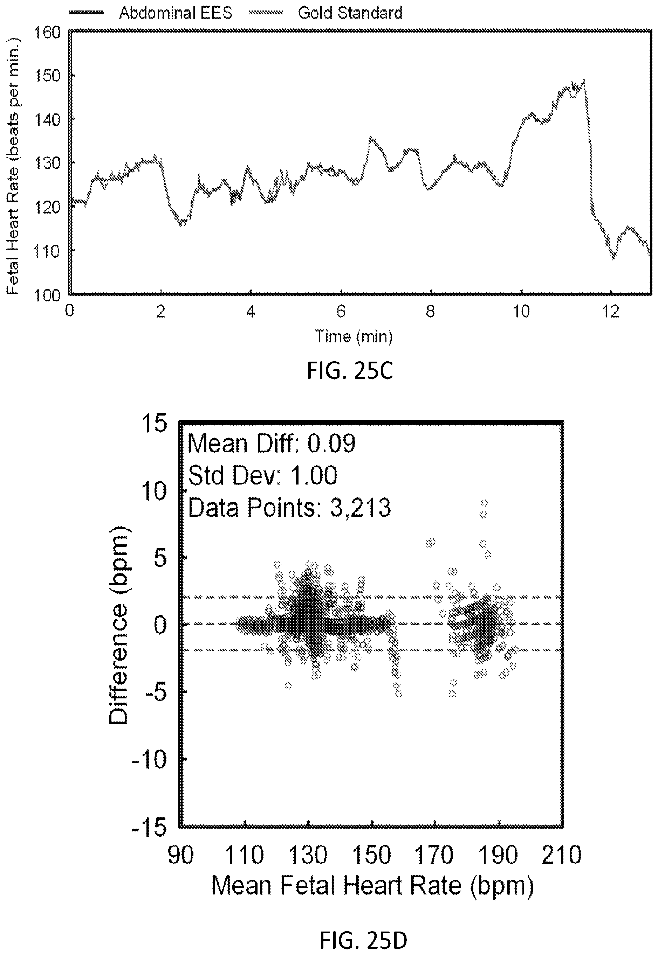

Continuous fetal heart rate (FHR) is commonly tracked in high resource settings during labor via wired Doppler ultrasound pucks strapped to the abdomen. The wireless abdominal sensor presented here generates an ultrasound waveform at a set frequency via an onboard transducer. The signal penetrates soft tissue and is reflected by abrupt changes in acoustic impedance. Upon reflection, the waves are altered to a different frequency with the Doppler Effect. The waves are then demodulated to the appropriate frequency for further signal processing. FIG. 25A represents the flow diagram of the algorithm to derive FHR via wireless ultrasound. First, the audible fetal phonocardiogram from the Doppler US is high pass filtered (f_{c} = 1 Hz), fed through a Hilbert transform algorithm and then scaled to obtain a positive envelope function. The envelope function is then segmented and processed with an autocorrelation function to identify peaks of recurring frequencies. The intervals of these frequencies are continuously updated and used to calculate FHR. FIG. 25B illustrates the collected raw Doppler signal from the abdominal sensor. There are two distinct peaks identifiable in the signal. The first peak "S1" represents closure of the tricuspid and mitral valves of the fetal heart, and the second peak "S2" represents closure of the pulmonary semilunar vales of the fetal heart. A sequence of the two peaks constitute one heartbeat of the fetus. To replicate the audible Doppler signal available in a gold standard tocodynamometer (GE Corometrics 250cx, GE Healthcare), the signal is normalized to a scale between [-1, 1] and encoded to the original sampling rate of 504 Hz. The result is an audio file (.WAV) which produces a sound representative of a fetal Doppler. Although intended for measuring maternal ECG, maternal HR and uterine contraction, the abdominal sensor is also capable for capturing incidental fetal electrocardiogram (fECG) in a subset of laboring women, which coincides with the fetal Doppler signal. Larger peaks can be identified as maternal ECG (mECG), and smaller peaks are indicative of fECG which are typically an order of magnitude lower in amplitude. For the sampled data, the R-to-R peak interval for mECG was averaged at about 635 ms, and the fECG R-to-R peak interval was averaged at about 344 ms. The R-to-R peak intervals translate to about 94 bpm and about 170 bpm for maternal and fetal HR, respectively.

FIGS. 25C-25D illustrate a FHR calculation between the abdominal sensor and gold standard Doppler US systems. The abdominal sensor is able to follow both expected fetal heart rate variability in a healthy fetus over time. A Bland-Altman comparing data from the Abdominal EES to the gold standard is presented in FIG. 35D for a subset of n=10 laboring women contributing 3,213 single datapoints. The mean difference is 0.1 beats per minute with a standard deviation of 1 beat per minute showing excellent agreement.

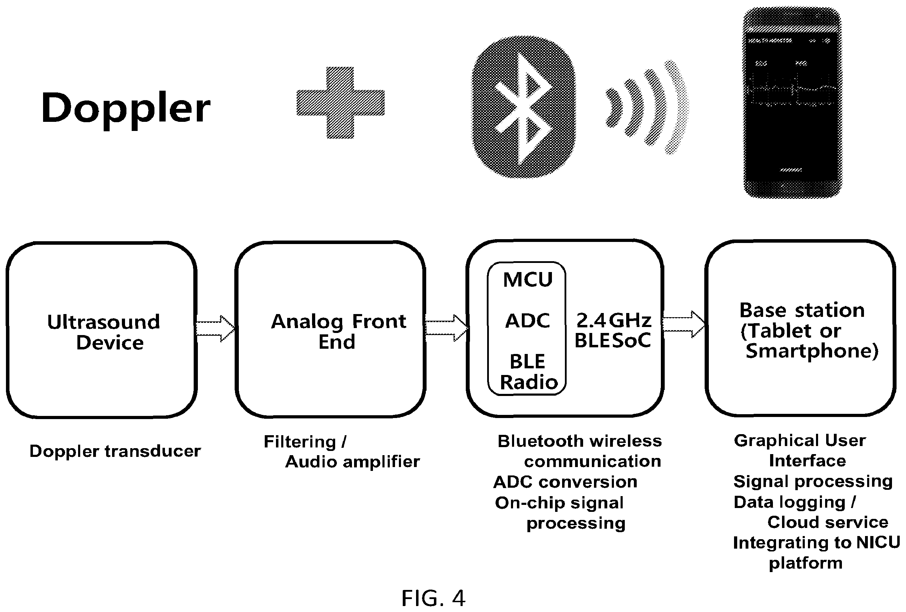

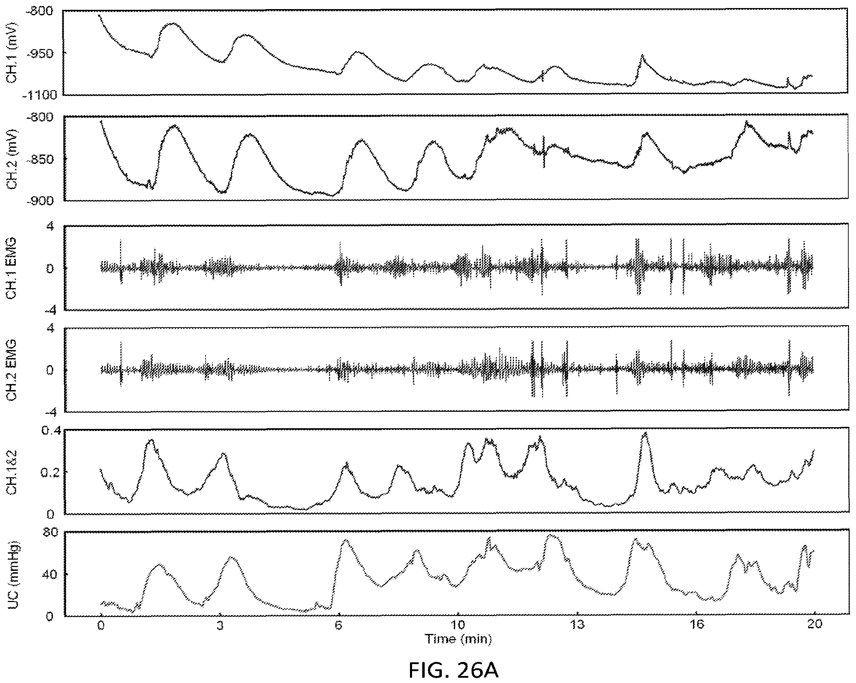

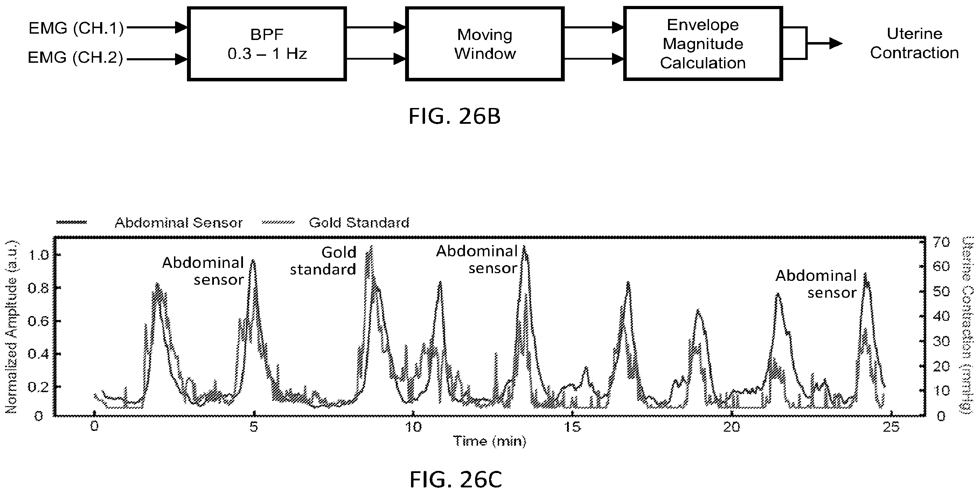

FIGS. 26A-26C illustrate the derivation of uterine contraction from the abdominal sensor's EMG capabilities. The physical tightening of the uterine muscles are sensed at the skin surface. As shown in FIG. 26A, the dual EMG signal obtained by both channels 1 and 2 is bandpass filtered (f_{c1} = 0.3 Hz, f_{c2} = 1 Hz). A moving window then searches for envelope magnitude detection, which is aggregated into a single waveform. The EMG contraction can be converted to an mmHg (pressure) with simple calibration. FIG. 4B illustrates a one-to-one contraction comparison between data retrieved from the abdominal sensor and a gold standard tocodynamometer (GE Corometrics 250cx, GE Healthcare). The abdominal sensor's capability of being able to consistently detect contractions throughout a laboring women's non-stress test session is demonstrated. Performance of the abdominal sensor was assessed by comparing peak counts with the gold standard system. Discernment of individual contractions was made by a board-certified obstetrician-gynecologist. FIG. 26C shows time series comparison of EMG derived uterine contractions from our abdominal sensor compared with a gold standard tocodynamometer.

### Clinical Validation and Deployments in Both High Resource and Low Resource Settings

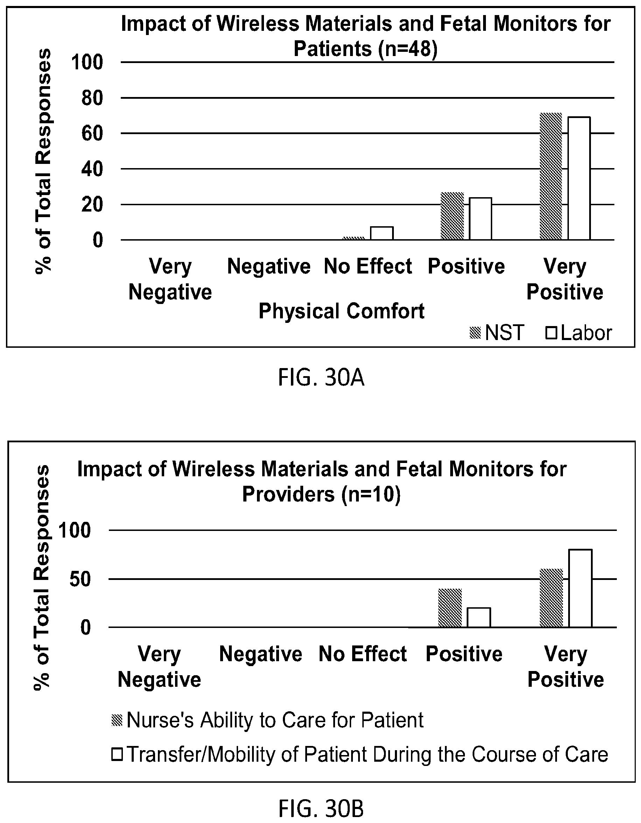

In this exemplary study, performance, feasibility, and usability from the sensors, both at the individual and population level, in a pilot cohort of 572 individuals were presented. 84% of these subjects were recruited in low resource settings (Lusaka, Zambia). In low resource settings, these sensors are used in a large, international, multicenter, single-arm, open-label clinical trial. The Limiting Adverse Birth Outcomes in Resource-Limited Settings (LABOR) study is documenting labor, delivery, and early postpartum in 15,000 mother-newborn pairs via detailed physiological data generated by these sensors along with laboratory and clinical data. The resulting datasets are used to generate new predictive algorithms to proactively manage laboring women to prevent adverse maternal and neonatal outcomes. All subjects provided informed consent with research protocols approved by Institutional Review Boards of Northwestern University (STU00205895). The final cohort recruited in the study included a wide range of ages (18-43), ethnicities, and ages from preterm to late term gestational ages (25-41 weeks). A cohort of pregnant women and obstetrical providers were surveyed (FIGS. 30A-30B). More than 85% of laboring women surveyed (n=48) indicate a positive or very positive experience with the wireless sensors. 100% of providers (n=10) indicate a positive or very positive experience with the wireless sensors. See Table 2 for demographic data on all subjects.

### Continuous Surrogates of Systolic Blood Pressure Derived from Pulse Arrival Time and Pulse Transit Time

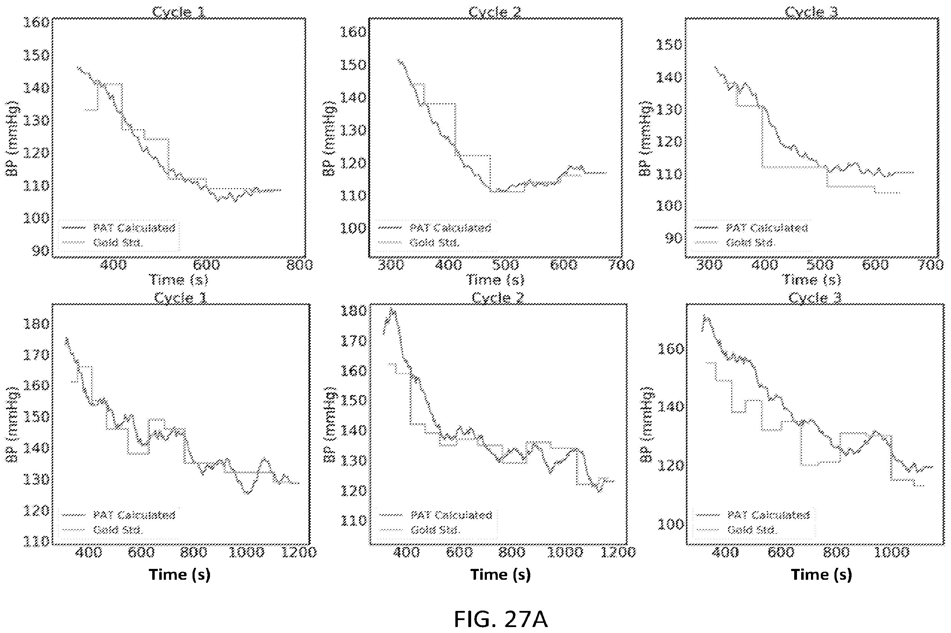

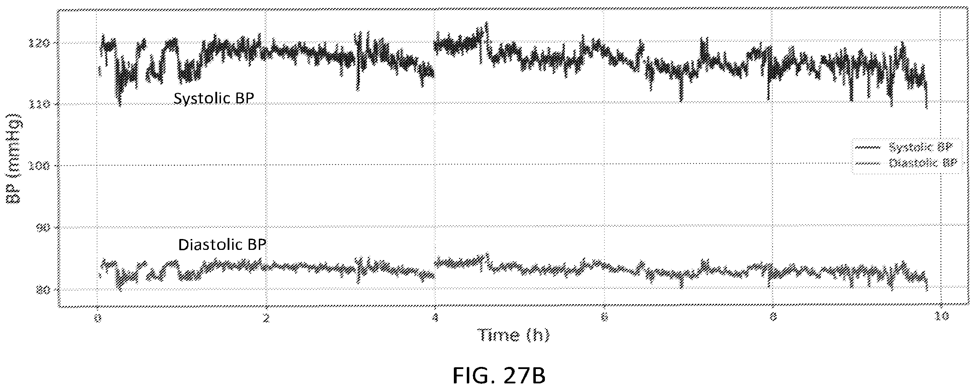

Hypertensive disorders of pregnancy (e.g., eclampsia) cause significant morbidity and mortality in both high resource and low resource settings. While the blood pressure is assessable with traditional sphygmomanometers, these systems are non-continuous and require clinical staff to deploy which may be limited in low income settings. Herein, the exemplary study demonstrates the derivation of a continuous surrogate of systolic blood pressure via PAT and PTT without the need for clinical staff to implement and record. The accuracy and performance of this method in a cohort of pediatric subjects were previously demonstrated. FIG. 27A demonstrates a high degree of agreement with traditional FDA-cleared sphygmomanometers capturing blood pressure at discrete time points with errors less than 10 mmHg. FIG. 27B demonstrates the utility of this measurement over a prolonged labor (10 hours) with the sensors after a single point calibration with a sphygmomanometer.

### Time Series Data of Vital Signs during Labor

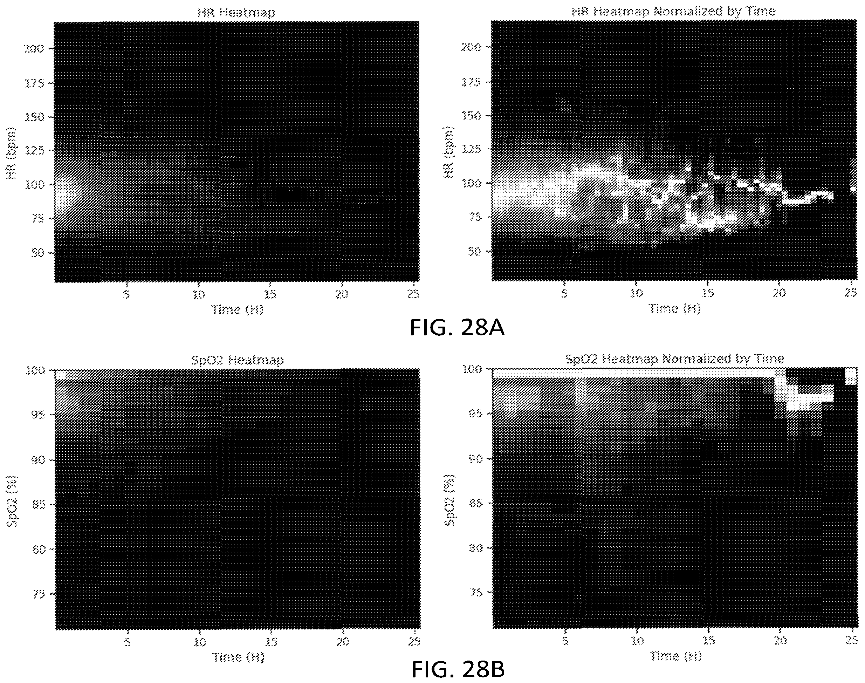

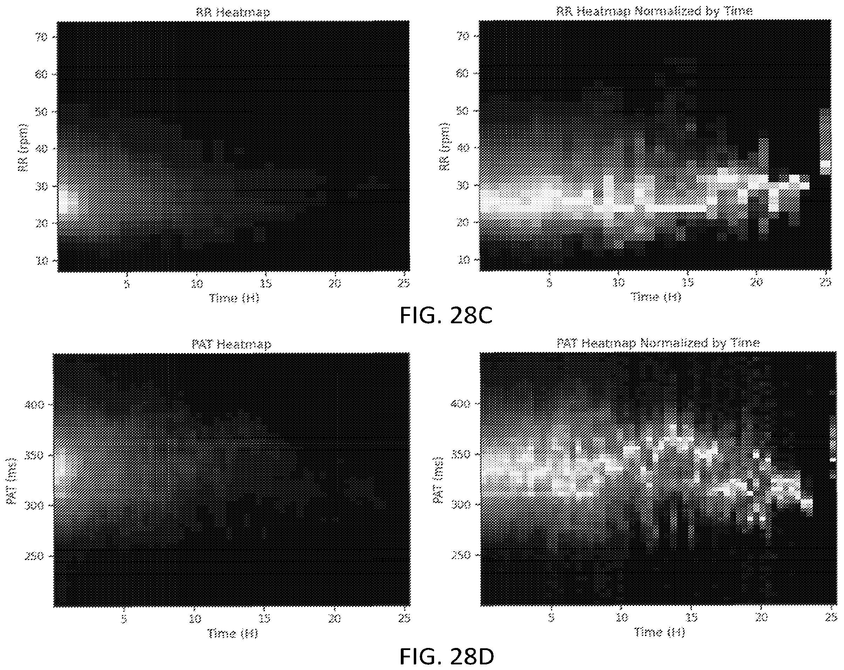

The ability to continuously track vital signs allows for detailed physiological profiling of a women's health status during the course of labor. FIGS. 28A-28D shows time series data of vital signs during labor. In the cohort of n=572 laboring subjects, heatmaps illustrating median vital signs for heart rate, SpO₂, respiratory rate, and PAT converted to BP are demonstrated (Table 2). We further strategy mean values based on body position - automatically classified by the system.

As most laboring periods did not extend beyond 5 hours, additional graphical plots were provided, where each vital signs are normalized on an hourly basis to illustrate expected vital sign parameters for women with prolonged labors. The future utility of this platform allows for automated identification for subjects and alerts to qualified healthcare providers who deviate from the norm in a time sensitive manner for heart rate, respiratory rate, blood oxygenation, and systolic blood pressure.

**Table 2: Mean vital signs for HR, SpO₂, RR, SBP, DBP, PAT, and chest and limb temperatures**

| **Mean** Posture \ Vital | HR (bpm) | SpO₂ (%) | RR (rbpm) | SBP (mmHg) | DBP (mmHg) | PAT (ms) | Chest Temp (°C) | Limb Temp (°C) |
|---|---|---|---|---|---|---|---|---|
| Hand-knees | 90.8 | 90.9 | 24.2 | 130.7 | 79.9 | 315.9 | 35.9 | 34.6 |
| High Fowler | 93.1 | 91.9 | 24.2 | 126.5 | 76.7 | 315.2 | 35.4 | 34.1 |
| Lateral | 93.9 | 90.6 | 25.7 | 125.9 | 76.1 | 308.4 | 35.6 | 34.5 |
| Supine | 91.2 | 91.6 | 25.3 | 128.1 | 77.5 | 314.2 | 35.5 | 34.2 |

### Real Time Tracking of Maternal Body Position during Labor

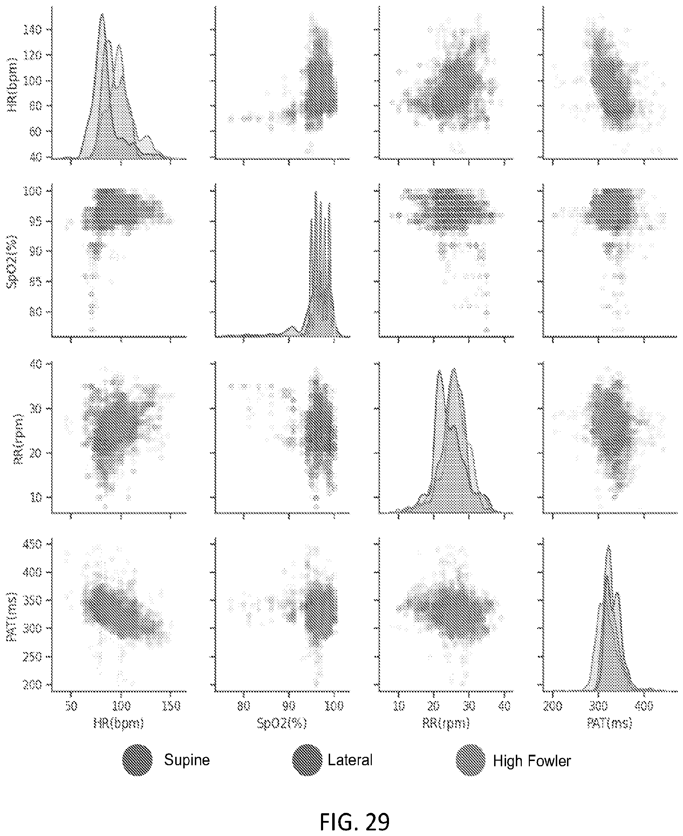

Maternal body positioning during labor is a common obstetrical intervention. FIG. 29 demonstrates the response of heart rate, blood oxygenation, respiratory rate, and pulse arrival time converted to BP categorized by different body positions (supine, lateral, hands and knees, and high Fowler). To the inventors' knowledge, this is the first monitoring system able to automatically categorize pregnancy body positions with comprehensive vital signs monitoring. This enables the future ability to quantify physiological responses to body position changes to optimize birth outcomes.

### Discussion

Currently, pregnancy monitoring is limited by systems that have undergone little innovation over the past several decades. Conventional cardiotocography (CTG) uses a large central monitor with wired probes placed on the maternal abdomen. The first probe is a Doppler sensor that measures the fetal heart rate and the second probe is a tocodynamometer that captures uterine contraction. The probes are connected by broad cables to the base unit and are further held in place by an elastic fabric bands circling the patient's waist. This limits a laboring women's mobility and comfort during labor. Assessing maternal vital signs such as the heart rate, the respiratory rate, the pulse oxygenation, and the blood pressure requires a new sensor wired to the body configured to another bulky base unit. Ultimately, evaluating the full spectrum of both maternal and fetal health requires a complex and expensive configurations of wired sensors and bulky base units. While there are newer platforms leveraging fetal electrocardiography, they still require bulky systems adhered to the body or wires to bulky base units. Furthermore, these systems are unable to be used in non-singleton pregnancies or preterm deliveries. Perhaps most notable, all of the aforementioned wireless devices focus data analytics on the fetus, and none incorporate maternal vital signs beyond uterine contractility. Existing pregnancy monitoring systems used in the high income settings are difficult to implement in low income settings where maternal and neonatal morbidity and mortality is the greatest.

The invention discloses, among other things, a new wireless monitoring platform/system that is able to provide comprehensive assessment of both the mother and fetus with compatibility across a wide range of mobile devices. The use of Doppler in the abdominal sensors allow us to monitor non-singleton pregnancies with multiple patches unlike newer fetal ECG systems. Beyond traditional vital signs, this system offers clinically relevant measurement modalities specific to pregnancy monitoring. This is the first reported system able to provide continuous body positioning categorization and continuous blood pressure assessments validated in pregnant women. The ability to track body position continuously and concurrently with maternal and fetal vital signs offers an objective way to assess physiological response. Altering maternal positioning during labor as a means of fetal resuscitation is common. For example, avoiding a supine position during labor prevents poor uteroplacental perfusion in the setting of maternal hypotension. Previous research efforts has also demonstrated that maternal position impacts maternal blood flow, labor progression, and patient comfort. The system according to embodiments of the invention is able to provide objective data on the impact of maternal body position on both maternal and fetal health. In some embodiments, the system also includes automated alerts to patients and providers for body position changes in instances where fetal or maternal distress is observed.

The ability to continuously monitor women after initial set up with automated alerts opens new opportunities for predictive algorithms without increasing provider burden. In both high and resource settings, the burden of clinical staff is of significant consideration. The system disclosed herein can be configured so as to alert providers only when thresholds of vital signs are crossed indicative of problems. Greater number of data sets with labeled clinical outcomes, generated by studies such as LABOR, offer the opportunity for more specific and sensitive warnings of maternal or fetal deterioration to support clinical decision making.

Finally, the sensors are able to seamlessly be used across the entire continuum of maternal, fetal, and neonatal care. A seamless transition from antepartum to laboring to postpartum surveillance may facilitate earlier detection of postpartum complications such as catastrophic hemorrhage leading to hemodynamic instability or hypertension secondary to preeclampsia. Neonatal mortality can also be tracked with the same sensors-our previous work has demonstrated accuracy, skin safety, and performance in premature neonates in both high resource and low resource settings.

Cost remains a critical consideration particularly in LMICs for perinatal monitoring technologies. Medical devices in low income settings often times are not sustainably used once donated. The key drivers of this failure is multifactorial including the unsustainable cost of maintenance, lack of skilled technicians to manage the equipment, out of date spare parts, and high consumable costs. The sensor systems presented here are designed to address these challenges. First, the sensors themselves are low cost (estimated less than $40 USDs at scale per sensor at scaled manufacturing) and fully reusable after a wireless recharge after a simple cleaning procedure. The reusability of the sensors dramatically lowers the variable cost of patient monitoring. The consumables to operate the system are also low cost constituting only a single use hydrogel adhesives and Latex-free fabric strap (estimated less than $0.25 per use). Finally, the system leverages a wide range of widely available mobile devices (Android or iOS) to display, store, and transmit data. By 2025, it is estimated that there will be nearly 700 million smartphones in Sub-Saharan Africa making these devices readily available at a low cost to operate the sensors for healthcare applications in even the most rural settings.

Medical monitoring is a cornerstone of modern prenatal and intrapartum care. Continuous data of maternal blood pressure, heart rate, oxygen saturation, uterine contractility, and fetal heart rate are potentially life-saving if acted on, but access is heavily dependent on a clinical infrastructure, the availability of skilled healthcare providers, and integration of disparate devices and their displays. The system we present here offers comprehensive and advanced measurement modalities with evidence of clinical utility, performance, and user preference in a substantial number of laboring women in both high resource and low resource settings. Technologies that marry maternal and fetal health in design and utility will improve access to data which can expedite diagnosis and necessary intervention at every point along the pathway to motherhood.

The foregoing description of the exemplary embodiments of the invention has been presented only for the purposes of illustration and description and is not intended to be exhaustive or to limit the invention to the precise forms disclosed. Many modifications and variations are possible in light of the above teaching.

Some references, which may include patents, patent applications and various non-patent literature publications, are cited and discussed in the description of this invention. The citation and/or discussion of such references is provided merely to clarify the description of the present invention and is not an admission that any such reference is "prior art" to the invention described herein.

### LIST OF REFERENCES

[1]. G. Sedgh, S. Singh, R. Hussain, Intended and unintended pregnancies worldwide in 2012 and recent trends. Stud Fam Plann 45, 301-314 (2014).
[2]. M. F. MacDorman, E. Declercq, H. Cabral, C. Morton, Recent Increases in the U.S. Maternal Mortality Rate: Disentangling Trends From Measurement Issues. Obstet Gynecol 128, 447-455 (2016).
[3]. Anonymous (Trends in maternal mortality: 2000 to 2017: estimates by WHO, UNICEF, UNFPA, World Bank Group and the United Nations Population Division. Geneva: World Health Organization; 2019.
[4]. R. W. Rochat, L. M. Koonin, H. K. Atrash, J. F. Jewett, Maternal mortality in the United States: report from the Maternal Mortality Collaborative. Obstet Gynecol 72, 91-97 (1988).
[5]. M. E. D'Alton et al., Putting the "M" back in maternal-fetal medicine: A 5-year report card on a collaborative effort to address maternal morbidity and mortality in the United States. Am J Obstet Gynecol 221, 311-317 e311 (2019).
[6]. B. Kodio et al., Levels and causes of maternal mortality in Senegal. Trop Med Int Health 7, 499-505 (2002).
[7]. K. Nagaya et al., Causes of maternal mortality in Japan. JAMA 283, 2661-2667 (2000).
[8]. V. Filippi, D. Chou, C. Ronsmans, W. Graham, L. Say, Levels and Causes of Maternal Mortality and Morbidity. Disease Control Priorities, Third Edition (Volume 2): Reproductive, Maternal, Newborn, and Child Health Published: April 2016. Pages: 51 - 70.
[9]. N. Vousden, H. L. Nathan, A. H. Shennan, Innovations in vital signs measurement for the detection of hypertension and shock in pregnancy. Reprod Health 15, 92 (2018).
[10]. S. Xu, A. Jayaraman, J. A. Rogers, Skin sensors are the future of health care. Nature 571, 319-321 (2019).
[11]. A. A. Boatin et al., Wireless Vital Sign Monitoring in Pregnant Women: A Functionality and Acceptability Study. Telemed J E Health 22, 564-571 (2016).
[12]. T. D. Knight MNM, Shakespeare J, Kenyon S, Kurinczuk JJ on behalf of MBRRACE-UK. (2017) Saving Lives, Improving Mother's Care- Lessons learned to inform maternity care from the UK and Ireland Confidential Enquiries into Maternal Deaths and Morbidity 2013-15.
[13]. L. G (2007) The Confidential Enquiry into Materna and Child Health (CEMACH). Saving Mothers Live's: reviewing maternal deaths to make motherhood safer - 2003-2005. The Seventh Report on Confidential Enquiries into Maternal Deaths in the United Kingdom.
[14]. T. Lavender, A. Hart, R. M. Smyth, Effect of partogram use on outcomes for women in spontaneous labour at term. Cochrane Database Syst Rev 10.1002/14651858.CD005461.pub3, CD005461 (2012).
[15]. Y. Ma et al., Relation between blood pressure and pulse wave velocity for human arteries. Proc Natl Acad Sci U S A 115, 11144-11149 (2018).
[16]. M. Gao, N. B. Olivier, R. Mukkamala, Comparison of noninvasive pulse transit time estimates as markers of blood pressure using invasive pulse transit time measurements as a reference. Physiol Rep 4 (2016).
[17]. Anonymous, Pulse Oximeters - Premarket Notification Submissions [510(k)s]: Guidance for Industry and Food and Drug Administration Staff. FDA. March 2013.
[18]. Anonymous, Cardiac Monitor Guidance (including Cardiotachometer and Rate Alarm) - Guidance for Industry. FDA Guidance. November 1998.
[19]. Anonymous, Limiting Adverse Birth Outcomes in Resource-Limited Settings (LABOR) study. ClinicalTrials.gov. NCT04102644. https://clinicaltrials.gov/ct2/show/NCT04102644. Accessed August 10th, 2020.
[20]. H. U. Chung et al., Skin-interfaced biosensors for advanced wireless physiological monitoring in neonatal and pediatric intensive-care units. Nat Med 26, 418-429 (2020).
[21]. R. Sameni, G. D. Clifford, A Review of Fetal ECG Signal Processing; Issues and Promising Directions. Open Pacing Electrophysiol Ther J 3, 4-20 (2010).
[22]. A. Wacker-Gussmann et al., Fetal cardiac time intervals in healthy pregnancies - an observational study by fetal ECG (Monica Healthcare System). J Perinat Med 46, 587-592 (2018).
[23]. P. R. Stone et al., Effect of maternal position on fetal behavioural state and heart rate variability in healthy late gestation pregnancy. J Physiol 595, 1213-1221 (2017).
[24]. G. Cito et al., Maternal position during non-stress test and fetal heart rate patterns. Acta Obstet Gynecol Scand 84, 335-338 (2005).
[25]. B. Carbonne, A. Benachi, M. L. Leveque, D. Cabrol, E. Papiernik, Maternal position during labor: effects on fetal oxygen saturation measured by pulse oximetry. Obstet Gynecol 88, 797-800 (1996).
[26]. S. M. Kinsella, A. Lee, J. A. Spencer, Maternal and fetal effects of the supine and pelvic tilt positions in late pregnancy. Eur J Obstet Gynecol Reprod Biol 36, 11-17 (1990).
[27]. O. Moraloglu et al., The influence of different maternal pushing positions on birth outcomes at the second stage of labor in nulliparous women. J Matern Fetal Neonatal Med 30, 245-249 (2017).
[28]. M. J. Guittier, V. Othenin-Girard, [Correcting occiput posterior position during labor: the role of maternal positions]. Gynecol Obstet Fertil 40, 255-260 (2012).
[29]. J. Roberts, L. Malasanos, C. Mendez-Bauer, Maternal positions in labor: analysis in relation to comfort and efficiency. Birth Defects Orig Artic Ser 17, 97-128 (1981).
[30]. A. S. Ginsburg et al., Evaluation of non-invasive continuous physiological monitoring devices for neonates in Nairobi, Kenya: a research protocol. BMJ Open 10, e035184 (2020).
[31]. B. Martinez et al., Agile Development of a Smartphone App for Perinatal Monitoring in a Resource-Constrained Setting. J Health Inform Dev Ctries 11 (2017).
[32]. Anonymous, Compton, B., D. M. Barash, J. Farrington, C. Hall, D. Herzog, V. Meka, E. Rafferty, K. Taylor, and A. Varghese. 2018. Access to medical devices in low-income countries: Addressing sustainability challenges in medical device donations. NAM Perspectives. Discussion Paper, National Academy of Medicine, Washington, DC. doi: 10.31478/201807a.
[33]. D. Radcliffe, Mobile in Sub-Saharan Africa: Can world's fastest-growing mobile region keep it up? ZDNEt. (2018).

## Claims

1. A sensor network for pregnancy monitoring of a subject, comprising:
a plurality of sensor systems time-synchronized to each other, wherein each sensor system is configured to be positioned on a respective region of the subject and comprises a sensor member configured to detect data associated with at least one of physiological parameters of the subject, and a Bluetooth low energy system-on-a-chip (BLE SoC) configured to process and transmit the detected data; and
a controller adapted in wireless communication with the plurality of sensor systems and configured to receive, from the plurality of sensor systems, and to process the detected data, and to display the physiological parameters,
wherein the physiological parameters comprise one or more of a maternal heart rate, a respiratory rate, a core body temperature, a peripheral temperature, a blood oxygenation, a blood pressure, a uterine contraction frequency and intensity, and a fetal heart rate,
wherein each of the plurality of sensor systems further comprises a power module coupled to the BLE SoC for providing power to said sensor system,
wherein each of the plurality of sensor systems further comprises:
a plurality of flexible and stretchable interconnects electrically connecting to a plurality of electronic components including the sensor member, the BLE SoC and the power module;
one or more foldable electronic boards, wherein the plurality of electronic components and the plurality of flexible and stretchable interconnects are disposed on the one or more foldable electronic boards; and
an elastomeric encapsulation layer at least partially surrounding the electronic components and the flexible and stretchable interconnects to form a tissue-facing surface attached to the subject and an environment-facing surface,
wherein the tissue-facing surface is configured to conform to a skin surface of the subject, wherein the elastomeric encapsulation layer is formed of a flame retardant material, and wherein the elastomeric encapsulation layer is a waterproof and biocompatible silicone enclosure.

2. The sensor network of claim 1, wherein the plurality of sensor systems comprises a first sensor system, a second sensor system and a third sensor system respectively configured to be positioned on an abdominal region, a chest region and a limb region of the subject, wherein the second sensor system is configured to be a central node, while the first and third sensor systems are configured to be peripheral nodes.

3. The sensor network of claim 2, wherein the first sensor system is configured to detect the fetal heart rate (FHR), the uterine contraction frequency and intensity, and/or the maternal heart rate, wherein the second sensor system is configured to detect the maternal heart rate, the core body temperature, and/or the respiratory rate (RR), and wherein the third sensor system is configured to detect the blood oxygenation and/or the peripheral temperature.

4. The sensor network of claim 3, wherein the sensor member of the first sensor system comprises an onboard wireless Doppler ultrasound (US) device for continuously acoustic detection of the FHR.

5. The sensor network of claim 4, wherein the first sensor system further comprises a piezoelectric transducer incorporated as a transmitter and receiver for a Doppler effect, wherein the piezoelectric transducer is tuned to resonate at about 3 MHz.

6. The sensor network of claim 4, wherein the sensor member of the first sensor system further comprises an electromyograph (EMG) sensor for detection of the uterine contraction frequency and intensity, electrocardiograph (ECG) sensor for simultaneously detection of a fetal ECG and a maternal ECG, and/or an accelerometer for measuring body position and/or physical activity, wherein the ECG sensor has at least two electrodes for ECG generation, wherein the at least two electrodes are adjustable in spacing.

7. The sensor network of claim 3, wherein the sensor member of the second sensor system comprises one or more of:
a motion-sensing unit for detecting seismocardiogram (SCG) and chest wall movements for respiratory rate (RR);
a thermometer for detecting the core body temperature; and
an ECG sensor for detecting the maternal heart rate,
wherein the motion-sensing unit comprises a 3-axial accelerometer, and/or an inertial measurement unit (IMU), wherein the accelerometer or the IMU is used with a motion artifact module to identify a vital sign as being subject to motion artifact and to correct of motion artifact.

8. The sensor network of claim 7, wherein the BLE SoC is configured to operably control the motion-sensing unit through one of a serial peripheral interface (SPI) communication protocol and an inter-integrated circuit (I²C) communication protocol, and to operably control the thermometer through the other of the SPI and the I²C communication protocols.

9. The sensor network of claim 3, wherein the sensor member of the third sensor system comprises a pulse oximetry for detecting blood oxygenation (SpO₂) and a thermometer for detecting the peripheral temperature, wherein the pulse oximetry comprises a light emitted diode (LED) and a photodetector.

10. The sensor network of claim 1,
wherein the power module comprises a power management integrated circuit (PMIC) coupled to the BLE SoC, a battery coupled to the PMIC for providing power to said sensor system, and a failure prevention element including a short-circuit protection component or a circuit to avoid battery malfunction.

11. The sensor network of claim 10, wherein the battery is a rechargeable battery,
wherein the power module further comprises a wireless charging module coupled to the PMIC for wirelessly charging the rechargeable battery, wherein the wireless charging module comprises a near-field communication (NFC) antenna.

12. The sensor network of claim 2, wherein each of the first and second sensor systems further comprises a biocompatible hydrogel adhesive for attaching said sensor systems on the respective region of the subject, wherein the biocompatible hydrogel adhesive is adapted such that signals from the subject are operably conductible to said sensor systems.

13. The sensor network of claim 1, wherein the controller is configured to perform at least one function of:
receiving and processing the detected data of the physiological parameters from the plurality of sensor systems;
displaying the processed data of the physiological parameters in real time;
transmitting the processed data of the physiological parameters to at least one of a patient database, a cloud server, and a mobile device;
continuously monitoring one or more critical parameters associating at least one vital sign; and notifying a practitioner or caregiver when a sensor aberrant signal output condition occurs; and
generating an alarm when an alarming vital sign reading condition in which the one or more of the critical parameters are out of pre-defined ranges occurs, and notifying a practitioner or caregiver of the alarm.

14. The sensor network of claims 4 and 13, wherein said processing the detected data of the physiological parameters comprises:
processing the output signals of the second and third sensor systems to determine a pulse arrival time (PAT) and a pulse transit time (PTT) so as to obtain the blood pressure;
eliminating a maternal ECG from the outputs of the first sensor system allowing for fetal ECG isolation; and/or
processing the output signals of the first and second sensor systems to subtract motion artifact in physiological signal calculations for respiratory rate and/or heart rate.

15. The sensor network of claim 13, wherein the controller is configured to further perform at least one function of:
assessing the physiological signs of a fetus and a mother during labor, based on the detected physiological parameters;
assessing the physiological signs of the fetus and the mother for women with high-risk obstetrical complications prior to labor; and
predicting for the onset of true labor from false labor.

16. The sensor network of claim 13, wherein the controller comprises a mobile device operable serving as a base station for Bluetooth communication, wherein the mobile device in bi-directionally wireless communication with the plurality of sensor systems, wherein the mobile device is a hand-held device or a portable device having a graphical user interface to display the physiological parameters.

## Patentansprüche

1. Sensornetzwerk zur Schwangerschaftsüberwachung einer Person, umfassend:
eine Mehrzahl von Sensorsystemen, welche miteinander zeitlich synchronisiert sind, wobei jedes Sensorsystem dazu eingerichtet ist, in einem jeweiligen Bereich der Person positioniert zu werden, und ein Sensorelement umfasst, welches dazu eingerichtet ist, Daten zu detektieren, welche mit wenigstens einem aus physiologischen Parametern der Person in Zusammenhang stehen, und ein Bluetooth Low Energy System-on-a-Chip (BLE SoC), welches dazu eingerichtet ist, die detektierten Daten zu verarbeiten und zu übertragen; und
eine Steuereinheit, welche in einer drahtlosen Kommunikation mit der Mehrzahl von Sensorsystemen ausgelegt ist und dazu eingerichtet ist, von der Mehrzahl von Sensorsystemen, die detektierten Daten zu empfangen und zu verarbeiten und die physiologischen Parameter anzuzeigen,
wobei die physiologischen Parameter eines oder mehre aus einer Herzfrequenz der Mutter, einer Atemfrequenz, einer Körperkerntemperatur, einer peripheren Temperatur, einer Blutsauerstoffsättigung, einem Blutdruck, einer Häufigkeit und Intensität von Gebärmutterkontraktionen und einer fetalen Herzfrequenz umfassen,
wobei jedes aus der Mehrzahl von Sensorsystemen ferner ein Leistungsmodul umfasst, welches mit dem BLE SoC zum Bereitstellen einer Leistung an das Sensorsystem gekoppelt ist,
wobei jedes aus der Mehrzahl von Sensorsystemen ferner umfasst:
eine Mehrzahl flexibler und dehnbarer Verbindungen, welche mit einer Mehrzahl elektronischer Komponenten, welche das Sensorelement, das BLE-SoC und das Leistungsmodul umfassen, elektrisch verbunden sind;
eine oder mehrere faltbare Elektronikplatinen, wobei die Mehrzahl elektronischer Komponenten und die Mehrzahl flexibler und dehnbarer Verbindungen an der einen oder mehreren faltbaren Elektronikplatinen angeordnet sind; und
eine elastomere Verkapselungsschicht, welche die elektronischen Komponenten und die flexiblen und dehnbaren Verbindungen wenigstens teilweise umgibt, um eine einem Gewebe zugewandte Fläche, welche an der Person angebracht ist, und eine einer Umgebung zugewandte Fläche zu bilden,
wobei die einem Gewebe zugewandte Fläche dazu eingerichtet ist, sich an eine Hautfläche der Person anzupassen, wobei die elastomere Verkapselungsschicht aus einem fammhemmenden Material gebildet ist, und wobei die elastomere Verkapselungsschicht eine wasserdichte und biokompatible Silikonhülle ist.

2. Sensornetzwerk nach Anspruch 1, wobei die Mehrzahl von Sensorsystemen ein erstes Sensorsystem, ein zweites Sensorsystem und ein drittes Sensorsystem umfasst, welche jeweils dazu eingerichtet sind, an einem abdominalen Bereich, einem Brustbereich und einem Extremitätenbereich der Person positioniert zu sein, wobei das zweite Sensorsystem dazu eingerichtet ist, ein zentraler Knoten zu sein, während das erste und das dritte Sensorsystem dazu eingerichtet sind, periphere Knoten zu sein.

3. Sensornetzwerk nach Anspruch 2, wobei das erste Sensorsystem dazu eingerichtet ist, die fetale Herzfrequenz (FHR), die Frequenz und Intensität von Uteruskontraktionen und/oder die Herzfrequenz der Mutter zu detektieren, wobei das zweite Sensorsystem dazu eingerichtet ist, die Herzfrequenz der Mutter, die Körperkerntemperatur und/oder die Atemfrequenz (RR) zu detektieren, und wobei das dritte Sensorsystem dazu eingerichtet ist, die Blutsauerstoffsättigung und/oder die periphere Temperatur zu detektieren.

4. Sensornetzwerk nach Anspruch 3, wobei das Sensorelement des ersten Sensorsystems eine integrierte drahtlose Doppler-Ultraschall-(US) Vorrichtung zur kontinuierlichen akustischen Detektion der FHR umfasst.

5. Sensornetzwerk nach Anspruch 4, wobei das erste Sensorsystem ferner einen piezoelektrischen Wandler umfasst, welcher als ein Sender und Empfänger für einen Doppler-Effekt integriert ist, wobei der piezoelektrische Wandler abgestimmt ist, um bei etwa 3 MHz in Resonanz zu schwingen.

6. Sensornetzwerk nach Anspruch 4, wobei das Sensorelement des ersten Sensorsystems ferner einen Elektromyographen- (EMG) Sensor zur Detektion der Frequenz und Intensität von Uteruskontraktionen, einen Elektrokardiographen-(ECG)-Sensor zur gleichzeitigen Detektion eines fetalen ECG und eines ECG der Mutter und/oder einen Beschleunigungsmesser zur Messung einer Körperposition und/oder einer körperlichen Aktivität umfasst, wobei der ECG-Sensor wenigstens zwei Elektroden zur ECG-Erzeugung aufweist, wobei die wenigstens zwei Elektroden im Abstand anpassbar sind.

7. Sensornetzwerk nach Anspruch 3, wobei das Sensorelement des zweiten Sensorsystems eines oder mehrere umfasst aus:
einer Bewegungsmesseinheit zum Detektieren eines Seismokardiogramms (SCG) und von Brustwandbewegungen für eine Atemfrequenz (RR);
ein Thermometer zum Detektieren der Körperkerntemperatur; und
einen ECG-Sensor zum Detektieren der Herzfrequenz der Mutter,
wobei die Bewegungsmesseinheit einen 3-Achsen-Beschleunigungsmesser und/oder eine Trägheitsmesseinheit (IMU) umfasst, wobei der Beschleunigungsmesser oder die IMU mit einem Bewegungsartefaktmodul verwendet wird, um ein Vitalzeichen als von Bewegungsartefakten betroffen zu identifizieren und ein Bewegungsartefakt zu korrigieren.

8. Sensornetzwerk nach Anspruch 7, wobei die BLE-SoC dazu eingerichtet ist, die Bewegungsmesseinheit über eines aus einem seriellen Peripherieschnittstellen-(SPI)-Kommunikationsprotokoll und einem Inter-Integrated-Circuit-(I²C)-Kommunikationsprotokoll funktionsfähig zu steuern und das Thermometer über das andere aus den SPI- und I²C-Kommunikationsprotokollen funktionsfähig zu steuern.

9. Sensornetzwerk nach Anspruch 3, wobei das Sensorelement des dritten Sensorsystems eine Pulsoxymetrie zum Detektieren einer Blutsauerstoffsättigung (SpO₂) und ein Thermometer zum Detektieren der peripheren Temperatur umfasst, wobei die Pulsoxymetrie eine Leuchtdiode (LED) und einen Fotodetektor umfasst.

10. Sensornetzwerk nach Anspruch 1, wobei das Leistungsmodul eine mit der BLE-SoC gekoppelte Leistungsmanagement-Integrierte-Schaltung (PMIC), eine mit der PMIC gekoppelte Batterie zum Bereitstellen von Leistung an das Sensorsystem und ein Ausfallverhinderungselement umfasst, welches eine Kurzschlussschutzkomponente oder eine Schaltung zum Verhindern einer Batteriestörung umfasst.

11. Sensornetzwerk nach Anspruch 10, wobei die Batterie eine wiederaufladbare Batterie ist, wobei das Leistungsmodul ferner ein drahtloses Lademodul umfasst, welches mit der PMIC zum drahtlosen Laden der wiederaufladbaren Batterie gekoppelt ist, wobei das drahtlose Lademodul eine Nahfeldkommunikation-(NFC)-Antenne umfasst.

12. Sensornetzwerk nach Anspruch 2, wobei jedes aus dem ersten und dem zweiten Sensorsystem ferner einen biokompatiblen Hydrogelkleber zum Anbringen der Sensorsysteme an dem jeweiligen Bereich der Person umfasst, wobei der biokompatible Hydrogelkleber derart ausgelegt ist, dass Signale von der Person funktionsfähig zu den Sensorsystemen leitbar sind.

13. Sensornetzwerk nach Anspruch 1, wobei die Steuereinheit dazu eingerichtet ist, wenigstens eine Funktion auszuführen aus:
Empfangen und Verarbeiten der detektierten Daten der physiologischen Parameter von der Mehrzahl von Sensorsystemen;
Anzeigen der verarbeiteten Daten der physiologischen Parameter in Echtzeit;
Übertragen der verarbeiteten Daten der physiologischen Parameter an wenigstens eines aus einer Patientendatenbank, einem Cloud-Server und einer mobilen Vorrichtung;
kontinuierliches Überwachen eines oder mehrerer kritischer Parameter, welche wenigstens ein Vitalzeichen zuordnen; und Benachrichtigen eines Arztes oder einer Pflegekraft, wenn ein abweichender Sensorausgabezustand auftritt; und
Generieren eines Alarms, wenn ein alarmierender Vitalzeichen-Messzustand auftritt, bei welchem der eine oder die mehreren kritischen Parameter außerhalb vordefinierter Bereiche liegen, und Benachrichtigen eines Arztes oder einer Pflegekraft über den Alarm.

14. Sensornetzwerk nach Ansprüchen 4 und 13, wobei die Verarbeitung der detektierten Daten der physiologischen Parameter umfasst:
Verarbeiten der Ausgabesignale des zweiten und dritten Sensorsystems, um eine Pulsankunftszeit (PAT) und eine Pulstransitzeit (PTT) zu bestimmen, um den Blutdruck zu erhalten;
Eliminieren eines ECG der Mutter aus den Ausgaben des ersten Sensorsystems, um eine Isolierung des fetalen ECG zu ermöglichen; und/oder
Verarbeiten der Ausgabesignale des ersten und zweiten Sensorsystems, um ein Bewegungsartefakt bei einer Berechnung physiologischer Signale für eine Atemfrequenz und/oder Herzfrequenz zu subtrahieren.

15. Sensornetzwerk nach Anspruch 13, wobei die Steuereinheit dazu eingerichtet ist, ferner wenigstens eine Funktion auszuführen aus:
Beurteilen der physiologischen Anzeichen eines Fötus und einer Mutter während Wehen auf der Grundlage der detektierten physiologischen Parameter;
Beurteilen der physiologischen Anzeichen des Fötus und der Mutter bei Frauen mit hochriskanten Geburtskomplikationen vor Wehen; und
Vorhersagen des Einsetzens echter Wehen gegenüber falschen Wehen.

16. Sensornetzwerk nach Anspruch 13, wobei die Steuereinheit eine mobile Vorrichtung umfasst, welche funktionsfähig als eine Basisstation für eine Bluetooth-Kommunikation dient, wobei die mobile Vorrichtung in bidirektionaler drahtloser Kommunikation mit der Mehrzahl von Sensorsystemen steht, wobei die mobile Vorrichtung eine handgehaltene Vorrichtung oder eine tragbare Vorrichtung ist, welche eine grafische Benutzerschnittstelle aufweist, um die physiologischen Parameter anzuzeigen.

## Revendications

1. Réseau de capteurs pour le suivi de grossesse d'un sujet, comprenant:
une pluralité de systèmes capteurs synchronisés temporellement entre eux, dans lequel chaque système capteur est configuré pour être positionné sur une région respective du sujet et comprend un élément capteur configuré pour détecter des données associées à au moins l'un de paramètres physiologiques du sujet, et un système sur puce Bluetooth Low Energy (BLE SoC) configuré pour traiter et transmettre les données détectées; et
un organe de commande adapté en communication sans fil avec la pluralité de systèmes capteurs et configuré pour recevoir, de la pluralité de systèmes capteurs, et pour traiter les données détectées, et pour afficher les paramètres physiologiques,
dans lequel les paramètres physiologiques comprennent un ou plusieurs parmi une fréquence cardiaque maternelle, une fréquence respiratoire, une température corporelle centrale, une température périphérique, une oxygénation sanguine, une pression sanguine, une fréquence et une intensité des contractions utérines, et une fréquence cardiaque fœtale,
dans lequel chacun de la pluralité de systèmes capteurs comprend en outre un module d'alimentation couplé au BLE SoC pour fournir de l'énergie audit système capteur,
dans lequel chacun de la pluralité de systèmes capteurs comprend en outre:
une pluralité d'interconnexions flexibles et extensibles assurant la connexion électrique avec une pluralité de composants électroniques incluant l'élément capteur, le BLE SoC et le module d'alimentation;
une ou plusieurs cartes électroniques pliables, dans lequel la pluralité de composants électroniques et la pluralité d'interconnexions flexibles et extensibles sont disposées sur les une ou plusieurs cartes électroniques pliables; et
une couche d'encapsulation élastomère entourant au moins partiellement les composants électroniques et les interconnexions flexibles et extensibles pour former une surface tournée vers le tissu fixée au sujet et une surface tournée vers l'environnement,
dans lequel la surface tournée vers le tissu est configurée pour se conformer à une surface cutanée du sujet,
dans lequel la couche d'encapsulation élastomère est formée d'un matériau ignifuge, et dans lequel la couche d'encapsulation élastomère est une enveloppe en silicone étanche et biocompatible.

2. Réseau de capteurs selon la revendication 1, dans lequel la pluralité de systèmes capteurs comprend un premier système capteur, un second système capteur et un troisième système capteur configurés respectivement pour être positionnés sur une région abdominale, une région thoracique et une région de membre du sujet, dans lequel le second système capteur est configuré pour être un nœud central, tandis que les premier et troisième systèmes capteurs sont configurés pour être des nœuds périphériques.

3. Réseau de capteurs selon la revendication 2, dans lequel le premier système capteur est configuré pour détecter la fréquence cardiaque fœtale (FCF), la fréquence et l'intensité des contractions utérines et/ou la fréquence cardiaque maternelle, dans lequel le second système capteur est configuré pour détecter la fréquence cardiaque maternelle, la température corporelle centrale et/ou la fréquence respiratoire (FR), et dans lequel le troisième système capteur est configuré pour détecter l'oxygénation sanguine et/ou la température périphérique.

4. Réseau de capteurs selon la revendication 3, dans lequel l'élément capteur du premier système capteur comprend un dispositif Doppler à ultrasons (US) sans fil embarqué pour la détection acoustique continue de la FCF.

5. Réseau de capteurs selon la revendication 4, dans lequel le premier système capteur comprend en outre un transducteur piézoélectrique incorporé comme émetteur et récepteur pour un effet Doppler, dans lequel le transducteur piézoélectrique est accordé pour résonner à environ 3 MHz.

6. Réseau de capteurs selon la revendication 4, dans lequel l'élément capteur du premier système capteur comprend en outre un capteur d'électromyographie (EMG) pour la détection de la fréquence et de l'intensité des contractions utérines, un capteur d'électrocardiographie (ECG) pour la détection simultanée d'un ECG fœtal et d'un ECG maternel, et/ou un accéléromètre pour la mesure de la position corporelle et/ou de l'activité physique, dans lequel le capteur ECG a au moins deux électrodes pour la génération d'un ECG, dans lequel les au moins deux électrodes sont réglables en espacement.

7. Réseau de capteurs selon la revendication 3, dans lequel l'élément capteur du second système capteur comprend un ou plusieurs de:
une unité de détection de mouvement pour détecter un sismocardiogramme (SCG) et les mouvements de la cage thoracique pour la fréquence respiratoire (FR);
un thermomètre pour détecter la température corporelle centrale; et
un capteur ECG pour détecter la fréquence cardiaque maternelle,
dans lequel l'unité de détection de mouvement comprend un accéléromètre triaxial et/ou une centrale inertielle (IMU), dans lequel l'accéléromètre ou l'IMU est utilisé avec un module d'artefact de mouvement pour identifier un signe vital comme étant sujet à un artefact de mouvement et pour corriger un artefact de mouvement.

8. Réseau de capteurs selon la revendication 7, dans lequel le BLE SoC est configuré pour commander de manière fonctionnelle l'unité de détection de mouvement par l'intermédiaire de l'un d'un protocole de communication d'interface périphérique série (SPI) et d'un protocole de communication inter-circuits intégrés (I²C), et pour commander de manière fonctionnelle le thermomètre par l'intermédiaire de l'autre des protocoles de communication SPI et I²C.

9. Réseau de capteurs selon la revendication 3, dans lequel l'élément capteur du troisième système capteur comprend un oxymètre de pouls pour détecter l'oxygénation sanguine (SpO₂) et un thermomètre pour détecter la température périphérique, dans lequel l'oxymètre de pouls comprenant une diode électroluminescente (DEL) et un photodétecteur.

10. Réseau de capteurs selon la revendication 1, dans lequel le module d'alimentation comprend un circuit intégré de gestion de l'alimentation (PMIC) couplé au BLE SoC, une batterie couplée au PMIC pour alimenter ledit système capteur, et un élément de prévention des défaillances incluant un composant de protection contre les courts-circuits ou un circuit pour éviter un dysfonctionnement de la batterie.

11. Réseau de capteurs selon la revendication 10, dans lequel la batterie est une batterie rechargeable, dans lequel le module d'alimentation comprend en outre un module de charge sans fil couplé au PMIC pour charger sans fil la batterie rechargeable, dans lequel le module de charge sans fil comprend une antenne de communication en champ proche (NFC).

12. Réseau de capteurs selon la revendication 2, dans lequel chacun des premier et second systèmes capteurs comprend en outre un adhésif hydrogel biocompatible pour fixer lesdits systèmes capteurs sur la région respective du sujet, dans lequel l'adhésif hydrogel biocompatible est adapté de telle sorte que les signaux provenant du sujet peuvent être conduits de manière fonctionnelle auxdits systèmes capteurs.

13. Réseau de capteurs selon la revendication 1, dans lequel l'organe de commande est configuré pour effectuer au moins une fonctions de:
recevoir et traiter les données détectées des paramètres physiologiques provenant de la pluralité de systèmes capteurs;
afficher en temps réel les données traitées des paramètres physiologiques;
transmettre les données traitées des paramètres physiologiques à au moins l'un d'une base de données patient, d'un serveur cloud et d'un dispositif mobile;
surveiller en continu un ou plusieurs paramètres critiques associés à au moins un signe vital; et informer un professionnel de santé ou un soignant lorsqu'un état de sortie de signal aberrant de capteur survient; et
générer une alarme lorsqu'un état de lecture de signe vital alarmant dans lequel les un ou plusieurs des paramètres critiques sont hors de plages prédéfinies survient, et informer un professionnel de santé ou un soignant de l'alarme.

14. Réseau de capteurs selon les revendications 4 et 13, dans lequel ledit traitement des données détectées des paramètres physiologiques comprend:
le traitement des signaux de sortie des second et troisième systèmes capteurs pour déterminer un temps d'arrivée du pouls (TAP) et un temps de transit du pouls (TTP) de manière à obtenir la pression sanguine;
l'élimination d'un ECG maternel des sorties du premier système capteur permettant l'isolement d'un ECG fœtal; et/ou
le traitement des signaux de sortie des premier et second systèmes capteurs pour soustraire un artefact de mouvement dans les calculs des signaux physiologiques pour la fréquence respiratoire et/ou la fréquence cardiaque.

15. Réseau de capteurs selon la revendication 13, dans lequel l'organe de commande est configuré pour effectuer en outre au moins une fonction de:
évaluer les signes physiologiques d'un fœtus et d'une mère pendant le travail, sur la base des paramètres physiologiques détectés;
évaluer les signes physiologiques du fœtus et de la mère pour les femmes présentant des complications obstétricales à haut risque avant le travail; et
prédire le déclenchement du travail véritable par rapport à un faux travail.

16. Réseau de capteurs selon la revendication 13, dans lequel l'organe de commande comprend un dispositif mobile servant de manière fonctionnelle de station de base pour la communication Bluetooth, dans lequel le dispositif mobile en communication sans fil bidirectionnelle avec la pluralité de systèmes capteurs, dans lequel le dispositif mobile est un dispositif tenu à la main ou un dispositif portable ayant une interface utilisateur graphique pour afficher les paramètres physiologiques.
